# EUROPEAN PATENT APPLICATION

(11) **EP 3 459 589 A1**
(43) Date of publication of application: **27.03.2019**
(21) Application number: 17306222.5
(22) Date of filing: 20.09.2017
(51) Int. Cl.: A61N 1/32

(54) **IONTOPHORESIS METHOD OF DELIVERING VITAMIN C THROUGH THE SKIN**

(71) Applicant: L'OREAL, 75008 Paris (FR)
(72) Inventor: BORDEAUX, Dominique, 94152 Chevilly LaRue (FR); CAZARES DELGADILLO, Jennyfer, 94152 Chevilly LaRue (FR); KALIA, Yogeshvar N., 1211 Geneva 4 (CH); DEL RIO SANCHO, Sergio, 1211 Geneva 4 (CH); SERNA JIMENEZ, Cesar, 1211 Geneva 4 (CH)
(74) Representative: Nony

(57) **Abstract**

The present invention relates to a iontophoresis method of delivering Vitamin C through the skin, the iontophoresis method comprising:
applying to the skin of a biological subject a composition comprising one or more of Vitamin C, Vitamin C derivatives, ions of Vitamin C, and ions of Vitamin C derivatives, and at least an anionic or non-ionic polymer,
applying simultaneously, successively or sequentially over time a selected current profile, either continuous direct current, pulsed current or a combination of both, from any device and/or support comprising at least one electrode to the skin, the continuous direct current, the pulsed current or the combination of both of a character and for a duration sufficient to transdermally deliver Vitamin C to the biological subject, and transporting different rates of Vitamin C across the skin in accordance to the selected current mode

## Description

The present invention relates to the field of skincare and/or the care of skin appendages. The term "skin and/or its appendages" is intended to mean in particular the skin, the mucous membranes, the lips, the scalp, the eyelashes, the eyebrows and the hair.

A subject of the invention is a cosmetic treatment method for the skin and/or appendages thereof, comprising at least one step consisting in applying at least one composition comprising at least Vitamin C or a derivative thereof to the skin. The method of the invention is other than therapeutic.

The present invention also relates to a topical cosmetic and/or dermatological composition comprising, in a physiologically acceptable medium, at least Vitamin C or a derivative thereof.

Vitamin C and its various forms (e.g., ascorbic acid, ascorbic acid salts, L-ascorbic acid, ascorbate, 2-oxo-L-threo-hexono-1,4-lactone-2,3-enediol, R)-3,4-dihydroxy-5-((S)-1,2-dihydroxyethyl)furan-2(5H)-one, oxidized forms ascorbic acid, anions of ascorbic acid dehydroascorbic, and the like) may have one or more beneficial uses. For example, vitamin C may act as an antioxidant against oxidative stress in humans. Vitamin C may also act as both a reducing agent and a free radical scavenger. One problem, however, is that vitamin C has a very low solubility in non-aqueous media. Additionally, if dissolved, vitamin C is easily oxidized and therefore loses function. Accordingly, some embodiments are directed to technologies and methodologies for transdermally delivering an aqueous active agent composition, such as vitamin C, to a biological subject.

Iontophoresis is the process of conducting an electrical current for the purpose of transporting charged molecules into the skin. The technique involves the application of a mild electrical current to a charged molecule by using a similarly charged electrode, as the molecule of interest to produce a repulsive effect that drives the charged molecules away from the electrode and into the skin. In some embodiments, the effect of simple ionization (electromigration) is the main mechanism by which iontophoresis produces its transport properties. However, there are additional mechanisms called electroosmosis and electroporation that are produced by an electrical current. Electroosmosis induces a flow of solvent that carries uncharged molecules in the anode-to-cathode direction.

In some embodiments, "iontophoresis" is the technique of using an electrical current to deliver molecules into the skin regardless of whether transport of the molecule is via electromigration or electroosmosis or electroporation.

Since many active molecules in skin care compositions have ionic forms, iontophoresis can be an effective tool for the administration of these active molecules. Vitamin C is also referred to as L-ascorbic acid, or by the systematic (International Union of Pure and Applied Chemistry) names, 2-oxo-L-threo-hexono-1,4-lactone-2,3-enediol or (*R*)-3,4-dihydroxy-5-((*S*)- 1,2-dihydroxyethyl)furan-2(5*H*)-one. Vitamin C is a negatively charged molecule at the physiological pH.

Accordingly, methods using electrical waveform stimuli for the administration of vitamin C into the skin are disclosed. In some embodiments, the administration of vitamin C is conducted on live, human beings. In some embodiments, the administration of vitamin C is conducted on any biological subject. In some embodiments, biological subjects include, but are not limited to, mammals, including human beings.

The efficacy of topical application of vitamin C is low as it has very low solubility in a non-aqueous medium, and if dissolved in aqueous media, vitamin C is easily oxidized and therefore loses function. In some embodiments, in cosmetics, vitamin C is used in compositions containing polymers to render the molecule stable. Although the presence of such substances usually plays a positive role in the formulation properties, they may also limit the efficient delivery of vitamin C into the skin. Accordingly, also disclosed are stable compositions formulated to increase the penetration of vitamin C, preferably in the ascorbic acid form, into skin by electrical currents, preferably by iontophoresis.

Analysis of skin penetration profiles suggests that galvanic current (also referred to as direct current (DC)) coupled with pulsed current significantly improved the topical transport of active agents. In some embodiments, such active agents include vitamin C in the ascorbic acid form that mainly includes the facilitated absorption phase in which the penetration of the molecule is efficiently enhanced by "iontophoresis." The skin penetration rate increases with respect to the type of formulation applied principally due to the impact of the formulation components, in addition to the physicochemical properties of vitamin C.

There is a need for improving the delivering of moderate-high size compounds, such as Vitamin C through the skin, without altering the skin.

There is a need to increase vitamin C quantity and delivery kinetics and to enhance speed of delivery into the skin; both compared to topical application.

There is a need to enhance further the permeability of Vitamin C through keratinous material, and in particular through the skin.

It has been surprisingly found that the use of at least an anionic or non-ionic polymer in the composition enables to further enhance its permeability for Vitamin C.

### Electrical method

In some embodiments, a method of delivering vitamin C, for example an aqueous vitamin C composition, through the skin, comprises
applying to the skin of a biological subject a composition comprising one or more of Vitamin C, Vitamin C derivatives, ions of Vitamin C, and ions of Vitamin C derivatives, and at least an anionic or non-ionic polymer,
applying a selected current profile, either continuous direct current, pulsed current or a combination of both, from any device and/or support comprising at least one electrode to a biological subject, the continuous direct current, the pulsed current or the combination of both of a character and for a duration sufficient to transdermally deliver vitamin C, for example an aqueous composition, to a biological subject, and transporting different rates of vitamin C across the skin in accordance to the selected current mode.

This method may allow significant changes in skin impedance and thus an increase permeability of the Vitamin C through keratinous material, and in particular through the skin.

Such increase in its diffusion may enable to optimize the amount of active agent needed for target treatments into different layers of the skin. There may be a higher amount of Vitamin C delivered into the skin at lower application times.

Moreover, thanks to the invention, there is an increase in the electrotransport trough keratinous materials of Vitamin C and its derivatives sensitive to electric or electromagnetic field, thanks to the polymer(s) present in the composition, depending on the choice of the polymers and the ratio of said polymers.

The method of the invention is cosmetic and non-therapeutic.

In an embodiment, the selected current profile is negative (also called cathodal iontophoresis). In another embodiment, the selected current profile could also be positive (also called anodal iontophoresis).

In some embodiments of the iontophoresis method, applying a selected current profile to a biological subject includes generating a continuous direct current stimulus having an average current density ranging from 0.001 mA/cm² to 0.5 mA/cm², preferably from 0.01 mA/cm² to 0.4 mA/cm², and more preferably from 0.05 mA/cm² to 0.3 mA/cm², and in particular from 0.1 mA/cm² to 0.5 mA/cm².

In some embodiments of the iontophoresis method, applying a selected current profile to a biological subject includes generating a continuous direct current stimulus having an average current density of 0.2 mA/cm².

The continuous direct current stimulus may be applied for a duration ranging from 15 seconds to 4 hours, preferably from 30 seconds to 120 minutes, preferably from 2 minutes to 50 minutes and more preferably from 3 minutes to 40 minutes.

In some embodiments, the direct current stimulus may be applied for duration of 5, 10 or 20 minutes.

The application of the current stimulus may be followed by an application of the composition without application of a current stimulus (passive diffusion), for a duration ranging from 30 seconds to 120 minutes, preferably from 2 minutes to 100 minutes and more preferably from 3 minute to 80 minutes, for example for a duration of 60 minutes.

In another embodiment, there is no passive diffusion.

In some embodiments of the iontophoresis method, applying a selected current profile to a biological subject includes generating a pulsed current having sinusoidal waveforms, non-sinusoidal waveforms, or combinations thereof.

In some embodiments of the iontophoresis method, applying a selected current profile to a biological subject includes generating a pulsed current having periodic square waveforms, rectangular waveforms, saw tooth waveforms, spiked waveforms, trapezoidal waveforms, triangle waveforms, or combinations thereof.

In some embodiments of the iontophoresis method, applying a selected current profile to a biological subject includes concurrently delivering the continuous direct current and the pulsed current and generating a pulsed current stimulus having an average current density ranging from 0.005 mA/cm² to 0.5 mA/cm², in particular from 0.05 mA/cm² to 0.5 mA/cm²; a pulse duration ranging from 100 microseconds to 500 microseconds, in particular from 200 microseconds to 300 microseconds; and a pulse frequency ranging from 1 Hertz to 500 Hertz, in particular from 100 Hertz to 300 Hertz.

### Composition

The composition may comprise one or more of Vitamin C, Vitamin C derivatives, ions of Vitamin C, and ions of Vitamin C derivatives present in amounts ranging from 0.01 % to 100 % by weight, preferably from 0.5 % to 60 % by weight, relative to the total weight of the composition. In an embodiment, the composition may comprise 5 % by weight of one or more of Vitamin C, Vitamin C derivatives, ions of Vitamin C, and ions of Vitamin C derivatives, relative to the total weight of the composition.

The composition may further comprise one or more anionic or non-ionic polymers present in amounts ranging from 0.01 % to 30 % by weight, preferably from 0.1 % to 10 % by weight, relative to the total weight of the composition.

The composition may further comprise a polar solvent present in an amount of at least 30 % by weight, preferably in an amount of at least 40 % by weight, and more preferably in an amount of at least 50 % by weight, relative to the total weight of the composition. The polar solvent may be present in an amount of at least 60 % by weight, preferably in an amount of at least 70 % by weight, relative to the total weight of the composition.

Chemical enhancers such as polar solvents in combination with iontophoresis may offer some advantages over using each technique separately. Their simultaneous use can be favorable in moderating the iontophoretic current regimen, and increase delivery efficiency of actives. In addition, they may also reduce skin irritation and improve safety of promoters.

The composition may comprise water present in an amount of at least 30 % by weight, in particular in an amount of at least 40 % by weight, and more preferably in an amount of at least 50 % by weight, relative to the total weight of the composition.

The iontophoresis composition of the invention may also comprise ethanol, in particular present in an amount of at least 1 % by weight, preferably in an amount of at least 5 % by weight, more preferably in an amount of 10 % by weight, relative to the total weight of the composition.

In an embodiment, the composition is deprived of any other anionic molecule which could otherwise be transdermally delivered through the skin. Those anionic molecules could indeed be transdermally delivered through the skin instead of vitamin C, and therefore reduce the amount of vitamin C which can be delivered through the skin.

The composition is for example deprived of any preservative, and/or of ethylenediamine tetra-acetic acid (EDTA).

The absence of said other anionic molecules may also enable to help reduce the risk of skin reactions and/or of deteriorate the biological activity of vitamin C and its derivatives.

In some embodiments of the iontophoresis method, the method further comprises transdermally delivering a composition, for example an aqueous composition, including, one or more of vitamin C, vitamin C derivatives, ions of vitamin C, and ions of vitamin C derivatives present in amounts ranging from 0.1 % to 20 % by weight; and one or more anionic or non-ionic polymers present in amounts ranging from 0.01 % to 10% by weight; and water present in an amount of at least 30 % by weight, relative to the total weight of the composition.

In some embodiments of the iontophoresis method, the method further comprises transdermally delivering an aqueous composition including,
one or more anionic or non-ionic polymers present in amounts ranging from 0.01 % to 10 % by weight, relative to the total weight of the composition; and
water present in an amount of at least 30 % by weight, relative to the total weight of the composition.

The composition may in particular be in the form of an aqueous cream or gel type.

Polymeric gels have several advantages over liquids like ease of manufacture, suitability with electrode design, deformability into skin contours, better stability and better occlusion. Moreover, the high proportions of water use in gel formulations provide an advantage in terms of electro-conductivity that will drive easily the active agent into the skin.

In some embodiments of the iontophoresis method, transdermally delivering vitamin C, in particular the aqueous active agent composition, includes generating a continuous direct current stimulus having an average current density ranging from 0.01 mA/cm² to 0.5 mA/cm²; and generating a pulsed current stimulus having an average current density ranging from 0.01 mA/cm² to 10 mA/cm²; a pulse duration ranging from 10 microseconds to 500 microseconds; and a pulse frequency ranging from 10 Hertz to 500 Hertz; the continuous direct current and the pulsed current of a duration sufficient to transdermally deliver an aqueous active agent composition to a biological subject.

In some embodiments of the iontophoresis method, the method comprises a step of measuring at least one of the temperatures of the skin, the impedance of the skin, and a pH of the composition.

In some embodiments of the iontophoresis method, the method comprises a step of measuring at least one of the viscosity, the conductivity, the color, the homogeneity, the microscopical aspect and/or the turbidity of the composition.

In some embodiments, skin preparation is performed to modify skin surface and skin caracteristics. Skin preparation may comprise cleansing, hydration, dermabrasion, and/or microperforation, this list not being limitative. The skin preparation may enable to homogenize skin surface, in order to lower impedance, to enable homogeneous treatment, and/or to ease active diffusion into skin (speed and quantity).

In some embodiments of the iontophoresis method, the application of current profile is reduced to a safety level when a measured value measured by one of the sensors exceeds a safety range or a safety value.

In some embodiments of the iontophoresis method, the method comprises a step of measuring the pH of the composition. When the measured pH exceeds a pH safety range, the application of current profile is switched to a safety level, for example a safety level less than 1V, such as 0,5V. The pH safety range may be pH 4 to 7. In some embodiment, when the measured pH exceeds a pH safety range, for example the range from 4 to 7, the device switches the polarity during a short time to enable to reequilibrate the pH.

In an embodiment, the pH is monitored to have a skin pH above 4.5, which is the isoelectrical point of the skin, in order to have the skin negatively charged.

In some embodiments of the iontophoresis method, the method comprises a step of measuring the impedance of the skin. When the measured impedance exceeds an impedance safety range, the application of current profile is reduced to a safety level to avoid adverse event. The safety level may be less than 1V, such as 0,5V. The impedance safety range may be 50 Ω to 1 MΩ.

In some embodiments of the iontophoresis method, the method comprises a step of measuring the temperature of the skin. When the measured temperature exceeds a temperature safety value, the application of current profile is switched to a safety level, for example less than 1V, such as 0,5V. The temperature safety value may be chosen less than 42°C.

In a preferred embodiment of the iontophoresis method, the method comprises the steps of:
- measuring the temperature of the skin, and
- measuring the impedance of the composition, and
- measuring the pH of the composition.
Then, the device is configured for treating the results and regulating the microcurrent and polarity.

### Polymers

For the purpose of the present invention, the term "anionic polymer" means any polymer comprising an overall charge at full deprotonation which is negative. An anionic polymer according to the invention may contain anionic groups and / or groups which can be ionized into anionic groups.

For the purpose of the present invention, the term "non-ionic polymer" means a neutral polymer exhibiting substantially no net charge. A non-ionic polymer may not contain ionic groups. A non-ionic polymer may be a polymer which cannot be ionized.

The anionic or non-ionic polymers according to the invention have a molecular weight ranging from 100 to 5,000,000 Daltons, preferably from 500 to 4,000,000 Daltons. They may have a molecular weight ranging from 1,000 to 3,000,000 Daltons.

The anionic polymer may have an anionic charge density of at least 0.7 meq / g, varying from 0.9 to 7 meq / g, and preferably from 0.9 to 4 meq / g.

The anionic charge density of a polymer corresponds to the number of moles of anionic charges per unit mass of polymer under the conditions where it is totally ionized. By "totally ionized", it is meant the state at which the different protonable groups of a polymer are all fully protoned. It can be determined by calculation if the structure of the polymer is known, that is to say the structure of the monomers constituting the polymer and their moral or weight proportion. It can also be determined experimentally by the Kjeldahl method.

A composition suitable for the invention may comprise one or more anionic polymers of different chemical nature and / or a different charge density.

The composition may have a conductivity of between 0.1 and 50 mS / cm, better between 0.5 and 25 mS / cm.

Thus, a composition may comprise one or more highly charged anionic polymers, that is to say filler greater than 4 meq / g and one or more anionic or non-ionic polymers with a low charge, that is to say a charge of less than 4 meq / g. In an embodiment, the composition may comprise an anionic polymer with a charge of around 0.1 meq / g.

The anionic polymers may be chosen from those containing primary, secondary, tertiary and/or quaternary amine groups.

In exemplary embodiments of the invention the polymers may be chosen among:
- ammonium polyacryloyldimethyl taurate, in particular the one marketed under reference HOSTACERIN AMPS of company CLARIANT,
- sodium carboxymethyl cellulose purified, in particular the one marketed under reference AQUASORB A 500 of company ASHLAND,
- sodium acryloyldimethyltaurate/VP crosspolymer (Aristoflex AVS), in particular the one marketed under reference ARISTOFLEX AVS of company CLARIANT,
- hydroxypropyl methyl cellulose (HPMC), in particular the one marketed under reference METHOCEL F 4 M PERSONAL CARE GRADE of company DOW CHEMICAL.

The composition may comprise hydroxypropyl methyl cellulose (HPMC) and/or ammonium polyacryloyldimethyl taurate and/or sodium acryloyldimethyltaurate/VP crosspolymer and/or sodium carboxymethyl cellulose purified.

The composition may comprise hydroxypropyl methyl cellulose (HPMC), for example in an amount ranging from 0.01 % to 10 % by weight, preferably of about 1.0 % by weight, relative to the total weight of the composition. A composition comprising 1% by weight of non-ionic polymer hydroxypropyl methyl cellulose (HPMC) in a mixture of water, propylene glycol, and ethanol was tested. This formulation provides an acid ascorbic enhancement ratio (diffusion by iontophoresis versus passive diffusion) of 10.9 compared to 2.0 from a reference formulation.

### Composition for the invention

According to a preferred embodiment, an anionic or non-ionic polymer used according to the invention is conveyed in a care and / or washing composition, in particular for keratinous materials.

A composition according to the invention is advantageously administered topically at the level of the target keratinous material.

Such a composition may be in the form of an aqueous, aqueous-alcoholic or oily solution, a solution or dispersion of the lotion or serum type, an emulsion of liquid or semi-liquid consistency of the milk type, obtained by dispersing of a fatty phase in an aqueous phase (O/W) or inversely (W/O), or a suspension, or emulsion, of soft, semisolid or solid consistency, of the type cream, aqueous or anhydrous gel, a microemulsion, a microcapsule, a microparticle, or a vesicular dispersion of ionic and / or nonionic type.

These compositions are prepared according to the usual methods.

These compositions may in particular constitute creams for the cleaning, protection, treatment or care, lotions, gels or foams for the care and cleaning of the skin, mucous membranes, scalp and / or keratinous materials such as the hair.

They may be used for the cosmetic and / or dermatological treatment of the skin, the mucous membranes, the scalp and / or keratinous materials such as the hair, in the form of solutions, creams, gels, emulsions, foams or in the form of compositions suitable for the use of an aerosol, for example also containing a propellant under pressure.

In a known manner, the galenical forms dedicated to topical administration may also contain conventional adjuvants in the cosmetic and / or dermatological field, such as thickeners, oils, waxes, preservatives, antioxidants, solvents, perfumes, fillers, UV filters and dyestuffs.

The amounts of these various adjuvants are those conventionally used in the field in question. These adjuvants, depending on their nature, can be introduced into the fatty phase and / or into the aqueous phase.

The composition of the invention may also advantageously contain water. The water may be a thermal and / or mineral water, in particular chosen from the Vittel water, the waters of the Vichy basin and the water from the Roche Posay. It may also be deionized water.

The water may be present in a content ranging from 1 to 99% by weight, relative to the total weight of the composition, preferably ranging from 10 to 95% by weight, preferably ranging from 15 to 95% by weight.

In some embodiments, the invention also relates to a cosmetic composition comprising one or more of Vitamin C, Vitamin C derivatives, ions of Vitamin C, and ions of Vitamin C derivatives, and at least an anionic or non-ionic polymer.

The cosmetic composition may comprise the anionic or non-ionic polymer in an amount ranging from 0.01% to 10 % by weight, preferably of about 1.0 % by weight, relative to the total weight of the composition.

In exemplary embodiments of the invention, the polymers may be chosen among:
- ammonium polyacryloyldimethyl taurate,
- sodium carboxymethyl cellulose purified,
- sodium acryloyldimethyltaurate/VP crosspolymer (Aristoflex AVS),
- hydroxypropyl methyl cellulose (HPMC).

The cosmetic composition may comprise hydroxypropyl methyl cellulose (HPMC) and/or ammonium polyacryloyldimethyl taurate and/or sodium acryloyldimethyltaurate/VP crosspolymer and/or sodium carboxymethyl cellulose purified.

The cosmetic composition may in particular comprise at least one of hydroxypropyl methyl cellulose (HPMC) and/or ammonium polyacryloyldimethyl taurate and/or sodium acryloyldimethyltaurate/VP crosspolymer.

The composition may also comprise ethanol present in an amount of at least 5 % by weight, preferably in an amount of at least 7 % by weight, more preferably in an amount of 10 % by weight.

In some embodiments, a cosmetic composition comprises one or more of vitamin C, vitamin C derivatives, ions of vitamin C, and ions of vitamin C derivatives present in amounts ranging from 0.1 % to 30 % by weight; one or more anionic or non-ionic polymers present in amounts ranging from 0.1 % to 30 % by weight; and water present in an amount of at least 20 % by weight; the iontophoresis composition having an aqueous phase that is at least 30% by weight relative to the total weight of the iontophoresis composition.

In some embodiments of the composition, the composition further comprises one or more anionic polymers present in amounts ranging from 0.01 % to 10% by weight; wherein the one or more of vitamin C, vitamin C derivatives, ions of vitamin C, and ions of vitamin C derivatives are present in amounts ranging from 0.1 % to 30 % by weight.

In some embodiments of the composition, the composition further comprises one or more non-ionic polymers present in amounts ranging from 0.01 % to 20% by weight; wherein the one or more of vitamin C, vitamin C derivatives, ions of vitamin C, and ions of vitamin C derivatives are present in amounts ranging from 0.01 % to 30 % by weight.

In some embodiments of the composition, the composition further comprises a pH ranging from 2 to 7.5. The pH of the composition may be above 4.5.

In some embodiments, when the measured pH exceeds a pH safety range, for example the range from 4 to 7, the device switches the polarity during a short time to enable to reequilibrate the pH.

### Iontophoresis kit

In some embodiments, the invention also provides an iontophoresis kit comprising:
- an iontophoresis composition including one or more of vitamin C, vitamin C derivatives, ions of vitamin C, and ions of vitamin C derivatives, and at least an anionic or non-ionic polymer, for example as described above, and
- an iontophoresis device for carrying the iontophoresis method as described above.

The iontophoresis composition may be as described above.

The invention also provides an iontophoresis kit comprising:
- an iontophoresis composition including one or more of vitamin C, vitamin C derivatives, ions of vitamin C and ions of vitamin C derivatives, and at least an anionic or non-ionic polymer, for example as described above, and
- an iontophoresis device for delivering the iontophoresis composition through the skin, configured for: applying a selected current profile, either continuous direct current, pulsed current or a combination of both. The current may be negative or positive.

The iontophoresis composition may be as described above.

The selected current profile may be as described above in relation with the method.

The composition may be an aqueous composition.

The iontophoresis kit may be configured such that vitamin C and water are already mixed in the composition when the composition is applied to the skin.

In some embodiments, the iontophoresis device may comprise at least one of a temperature sensor, an impedance sensor, and a pH sensor. The iontophoresis device may comprise at least two of a temperature sensor, an impedance sensor, and a pH sensor. In an embodiment, the iontophoresis device may comprise a temperature sensor, an impedance sensor, and a pH sensor.

The device may be configured such that the application of current profile is reduced to a safety level when a measured value measured by one of the sensors exceeds a safety range or a safety value.

In a preferred embodiment, the method above enables to reduce the spots on the hands and/or on the face and/or on the neck and/or décolleté, for example age spots and/or sunspot and/or freckles and/or spots due to a disease. Then, the above method enables the depigmentation of the skin, especially in zones where the pigmentation is initially too high. After several uses or a single use of the method of the invention, the color of the skin is more uniform and homogeneity is increased.
In another embodiment, the method is used to treat winkles and ageing signs, to improve smoothness, quality of skin and appearance of the skin.
In another embodiment, the method is used to minimize skin anti-aging, and/or pigmentation, and/or volume, and/ or sagging wrinkle, and/or event tone and/or spots, and/or to improve firmness, and/or radiance, and/or smoothness, and/or softness of the skin. The method of the invention may be associated with the application of active agents associated to electrical current, in particular microcurrent (µcurrent).

### DESCRIPTION OF THE DRAWINGS

The foregoing aspects and many of the attendant advantages of the disclosed subject matter will become more readily appreciated as the same become better understood by reference to the following detailed description, when taken in conjunction with the accompanying drawings, wherein:
FIGURE 1 is a schematic illustration of an iontophoresis device;
FIGURE 2 is a plot of an electrical waveform stimulus in accordance with one embodiment;
FIGURE 3 is a plot of an electrical waveform stimulus in accordance with one embodiment;
FIGURE 4 is a plot of an electrical waveform stimulus in accordance with one embodiment;
FIGURE 5 is a flow diagram of a method in accordance with one embodiment;
FIGURE 6 is a flow diagram of a method in accordance with one embodiment.
FIGURE 7a to 7c are diagrams comparing the efficacy of different compositions.

### DETAILED DESCRIPTION

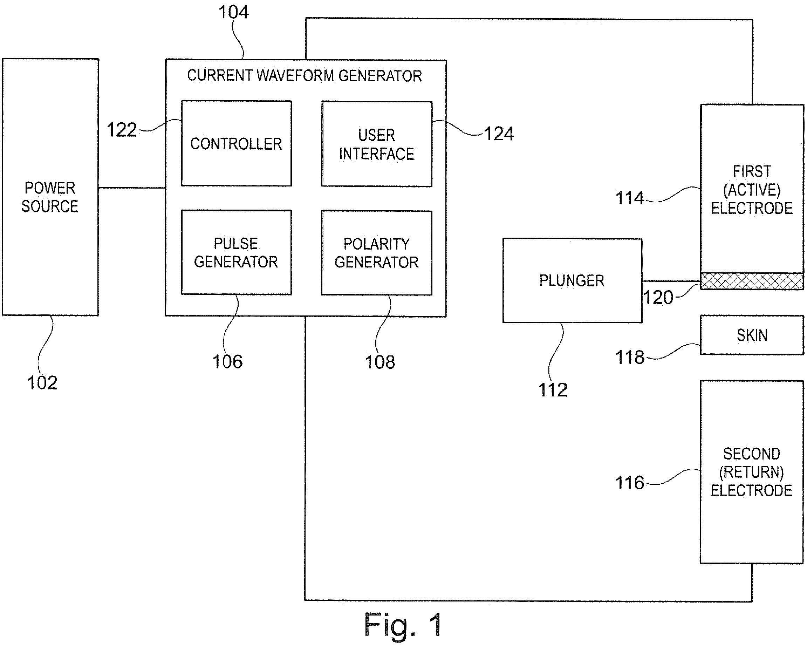

Referring to FIGURE 1, an iontophoresis device and electrode assembly is schematically illustrated. In some embodiments, the iontophoresis device and electrode assembly includes a power source 102, a current waveform generator 104, a first (active) electrode 114, a second (counter or return) electrode 116, a plunger 112, and a reservoir 120 at the end of the first electrode 114. It is to be appreciated that iontophoresis devices are available with additional features or fewer features. Therefore, other devices may include many other components that are not being shown or exclude some of the components that are shown. The purpose of FIGURE 1 is to illustrate and describe some of the major functional components of an iontophoresis device used to carry out one or more of the various embodiments of the methods for the application of vitamin C compositions or other active agent compositions disclosed herein. It is to be appreciated that implicit and inherent in FIGURE 1 is the circuitry used to carry out the functions of the iontophoresis device. In some embodiments, the iontophoresis device is packaged as a hand-held device that is suitable for carrying in one hand during treatment. Treatment using hand-held devices includes constantly moving the iontophoresis device over the skin so that the active electrode 114 is moved across the surface of the skin while making contact. In some embodiments, the iontophoresis device is packaged as a stationary desktop device, and the active electrode 114 is stationary and applied to a single location on the skin, such as through an adhesive. The major functional components of the iontophoresis device will now be described.

In some embodiments, the iontophoresis device includes a power source 102. A suitable power source would be any power source that can generate electrical current to power the various other circuits and devices. In some embodiments, a battery is used as the power source. Additionally, in some embodiments, an alternating power source coupled to a transformer can be connected to the power source 102. In some embodiments, the iontophoresis device is plugged into a wall socket. In some embodiments, the power source 102 produces continuous direct current. In some embodiments, circuitry is used to generate electrical waveforms other than continuous direct current. The power source 102 has a negative pole and a positive pole. Generally, negative polarity will be applied to the first electrode 114. However, through circuitry and electrical devices, such as switches, the polarities of the first 114 and second 116 electrodes can be reversed momentarily to achieve pulse and alternating current waveforms.

In some embodiments, the power source 102 is connected to the current waveform generator 104. The current waveform generator 104 functions to generate various types of current waveforms. In some embodiments, the current waveform generator 104 generates the waveforms through hardware and software, such as circuitry, described herein. In some embodiments, the waveform generator 104 includes circuitry operably coupled to an electrode assembly, and the circuitry is configured to concurrently generate at least a continuous direct current stimulus and a pulsed current stimulus to the same electrode, the continuous direct current stimulus and the pulsed current stimulus of a character and for a duration sufficient to deliver a cosmetic composition to a biological subject. The current generator 104 is connected to the first 114 and the second 116 electrodes and is able to apply a potential across the electrodes to supply electrical current stimuli in various waveforms described herein. Toward that end, the current waveform generator 104 includes a pulse generator 106 and a polarity generator 108. In some embodiments, functionally, the pulse generator 106 generates pulses of current of controlled amplitude and duration. In some embodiments, functionally, the polarity generator 108 controls the polarity at the first electrode 114 and second electrode 116. In some embodiments, the polarity generator 108 maintains the polarities of the first 114 and second 116 electrodes constant. In some embodiments, the polarity generator 108 applies zero polarity to the first and second 116 electrodes. In some embodiments, the polarity generator 108 applies the polarity to the first and second 116 electrodes in pulses at a predefined rate and duration. In some embodiments, the polarity generator 108 reverses the polarities of the first 114 and second 116 electrodes at a predefined rate or interval. In some embodiments, the polarity generator 116 is configured to apply constant polarity, pulses, or reverse polarity for predefined durations, sequentially or in any order.

In some embodiments, the iontophoresis device will include a controller 122. In some embodiments, functionally, the controller 122 will receive input from the user interface 124 and, in conjunction with the pulse generator 106 and the polarity generator 108, control the current density waveforms at the specifications input by the user/operator. In some embodiments, the controller 122, pulse generator 106, and polarity generator 108 are implemented in hardware components, such as analog circuitry, digital circuitry, microprocessors, or combinations thereof, or software components.

In some embodiments, the controller 122 has instructions for guiding a user to input the various parameters depending on the waveform stimulus that is to be applied. The user interface 124 can prompt the user for the information. In some embodiments, the controller 122 will request the user to select the stimulus output from a constant (DC) value, a pulse wave, or both a constant value and a pulse wave for the current density stimulus output waveform. Once the controller 122 receives input of the one or more stimulus wave types, the controller 122 prompts the user on the user interface 124 for parameters corresponding to the selected wave output type or types.

In some embodiments, the iontophoresis device includes the user interface 124. In some embodiments, functionally, the user interface 124 is for entry of data relating to the waveform types to be applied as electrical stimuli. In some embodiments, the user interface 124 includes an alphanumeric keyboard and display. In some embodiments, the user interface 124 includes directional arrow buttons and an enter button to enter data into memory. In some embodiments, the alphanumeric keyboard is implemented as a touch screen display. In some embodiments, the input of wave parameters is through the use of text boxes. In some embodiments, the input of wave parameters is through the use of scrolling menus.

Regardless of the manner of data entry, in some embodiments, the user interface 124 communicates a variety of prompts for the user to input information. In some embodiments, the user interface 124 prompts the user to input the duration of the treatment step. In some embodiments, the duration of the treatment step is the sum of time of the application of an electrical current of any one or more wave types and includes the time when electrical current is off for pulse waves. For example, a pulse wave set with a duty cycle of 50% has the electrical current off during 50% of the time, meaning that each pulse is one-half of each pulse cycle. However, the treatment duration will include the off period of the pulse cycle.

In some embodiments, the user interface 124 provides options to allow the user to input whether the current density output will be continuous direct current, pulse current, alternating pulse current, or any combination, and the duration of each wave type. In some embodiments, the user interface 124 prompts the user whether different wave types are superimposed on each other. For example, a pulse wave can be superimposed on a continuous direct current wave. In some embodiments, the user interface 124 prompts the user whether different wave types are combined in sequence. In some embodiments, the user interface 124 prompts the user to input the current density values to output for each wave type. In some embodiments, the current density is input as average current density, root mean square current density, or peak current density. In some embodiments, the user interface 124 prompts the user to input the polarity at the first electrode 114, including positive, negative, or both for alternating, with the corresponding constant or pulsed current output. In some embodiments, the user interface 124 prompts the user to input the electrodes' cross-sectional areas. In some embodiments, the user interface 124 prompts the user to input skin temperature. In some embodiments, the user interface 124 prompts the user to input the frequency of pulses, the maximum and minimum amplitudes of pulses, and the duration of pulses. In some embodiments, the user interface 124 prompts the user to input the % duty cycle of unidirectional pulses. In some embodiments, the user interface 124 prompts the user to input the % duty cycle of respective bipolar pulses. In some embodiments, the user interface 124 prompts the user to specify the time between pulses. In some embodiments, the user interface 124 prompts the user to specify the duration of a wave packet (wave train), and the frequency of the wave packets. In some embodiments, the user interface 124 prompts the user to specify the number of pulses in a wave packet (wave train). In some embodiments, the user interface 124 prompts the user to input the pulse wave shape, including periodic square waveforms, rectangular waveforms, saw tooth waveforms, spiked waveforms, trapezoidal waveforms, or triangle waveforms. In some embodiments, the user interface 124 prompts the user to input the treatment area. In some embodiments, the user interface 124 prompts the user to specify whether to apply alternating negative and positive pulses. In some embodiments, the user interface 124 prompts the user to specify whether pulses are to be unipolar or bipolar.

A unipolar pulse means that pulsed electrical current travels in one direction. A bipolar pulse means that pulsed electrical current travels in two directions or reverses directions. In some embodiments, the user interface 124 prompts the user to specify a ratio of negative to positive pulses or a ratio of positive to negative pulses. In some embodiments, the user interface 124 prompts the user whether to combine any continuous direct current waveform with any pulse waveform to provide two or more different waveforms concurrently. In some embodiments, the user interface 124 prompts the user to apply two or more waveforms concurrently, synchronously, sequentially, or alternately. In some embodiments, the user interface 124 prompts the user to input the duration of each waveform and the cycle time of each waveform if the waveforms alternate. In some embodiments, the user interface 124 prompts the user for pulse interval duration.

In some embodiments, when using two or more waveforms in a single treatment, the user interface 124 prompts the user to specify the treatment durations of the different waveforms, and how often each waveform cycles. In some embodiments when continuous direct current is used concurrently with a pulse wave, the user interface 124 prompts the user to specify the parameters of the pulse wave.

In some embodiments, the controller 122 carries out logic routines to direct the user interface 124 to present to the user the appropriate prompts so that the wave type information is entered. The controller 122 then uses the wave type parameters to generate, via the circuitry, including but not limited to the pulse generator 106 and the polarity generator 108, the appropriate stimulus wave according to the user entered parameters.

In some embodiments, the first electrode 114, also referred to as the active electrode, is connected to the power source 102 through the waveform generator 104. The first electrode 114 includes a reservoir 102 on the end thereof to hold a vitamin C composition or any active agent composition. In some embodiments, the reservoir 120 is a hollow indentation on the end of the first electrode 114, wherein the reservoir is used to contain a gel or gel-like vitamin C composition. Alternatively, in some embodiments, the reservoir 120 is an absorbent material to contain the vitamin C composition. In some embodiments, generally, the design of the first electrode 114 contemplates the first electrode 114 having negative polarity. When the iontophoresis device is packaged as a hand-held device, the first electrode 114 is provided with a type of roll-on applicator, such as a ball and socket fed by the plunger 112.

The skin 118 represents the load on the system.

In some embodiments, the second electrode 116, also referred to as the counter or return electrode, is connected to the power source 102 through the waveform generator 104. The polarity of the second electrode 116 is maintained by the waveform generator 104 to be the opposite of the polarity of the first electrode 114. In some embodiments, generally, the design of the second electrode 116 contemplates the second electrode 116 having positive polarity. In some embodiments, the second electrode 116 is a hand-held electrode that is held by the person receiving the treatment. In other embodiments, the second electrode 116 has an insulated cover and an exposed tip so that the second electrode is held by the device user and applied by the user on the skin of the person receiving the treatment.

In some embodiments, the reservoir 120 on the first electrode 114 is connected to a plunger 112. In some embodiments, functionally, the plunger 112 replenishes the vitamin C composition or any active agent composition in the reservoir 120. In some embodiments, the plunger 112 includes a piston and push pod within a cylindrical container. In some embodiments, the plunger 112 can be operated by a trigger mechanism that is operated during treatment by the user of the iontophoresis device.

In some embodiments, an iontophoresis device includes active (donor) and return (counter) electrode assemblies, a skin contacting layer, and an active agent layer. In some embodiments, an iontophoresis device includes active and return electrode assemblies having a multi-laminate construction. In some embodiments, an iontophoresis device includes electrode assemblies with a multi-laminate construction configured to deliver an active agent composition through passive diffusion or iontophoresis.

In some embodiments, an iontophoresis device includes active and return electrode assemblies, each formed by multiple layers of polymeric matrices. In some embodiments, an iontophoresis device includes electrode assemblies having a conductive resin film electrode layer, a hydrophilic gel reservoir layer, an aluminum or silver foil conductor layer, and an insulating backing layer.

In some embodiments, an iontophoresis device comprises an iontophoresis patch design. In some embodiments, an iontophoresis device comprises an iontophoresis multi-laminate design. In some embodiments, an iontophoresis device comprises an iontophoresis face mask design. In some embodiments, an iontophoresis device comprises an iontophoresis flexible substrate design.

In some embodiments, an electrode assembly includes at least one electrode and one or more compositions with an agent stored in a reservoir such as a cavity, a gel, a laminate, a membrane, a porous structure, a matrix, a substrate, or the like. Non-limiting examples of active agents include electrically neutral agents, molecules, or compounds capable of being delivered via electro-osmotic flow. In some embodiments, neutral agents are carried by the flow of, for example, a solvent during electrophoresis. In some embodiments, an iontophoresis device includes an electrode and a reservoir containing an effective amount of a vitamin C composition or any agent composition.

In some embodiments, a reservoir includes any form or matter employed to retain an element, a composition, a compound, active agent, a pharmaceutical composition, and the like, in a liquid state, solid state, gaseous state, mixed state or transitional state. In some embodiments, a reservoir includes one or more ion exchange membranes, semipermeable membranes, porous membranes or gels capable of at least temporarily retaining an element, a composition, a compound, active agent, a pharmaceutical composition, electrolyte solution, and the like.

In some embodiments, a reservoir includes one or more cavities formed by a structure. In some embodiments, a reservoir serves to retain an element, a composition, a compound, active agent, a pharmaceutical composition, electrolyte solution, and the like prior to the discharge of such by electromotive force or current into a biological interface. In some embodiments, an electrode assembly includes one or more ion exchange membranes that may be positioned to serve as a polarity selective barrier between the active agent reservoir and a biological interface.

In some embodiments, an electrode assembly includes an electrode and a reservoir containing an effective amount of a vitamin C composition or any aqueous active agent composition.

In some embodiments, the iontophoresis device includes circuitry that is coupled to the active electrode assembly, wherein the circuitry is configured to generate at least current stimuli from pulsed or continuous in a concurrent manner. In some embodiments, the circuitry is included in the iontophoresis device and the current waveform generator 104 illustrated in FIGURE 1. In some embodiments, circuitry applies a potential across the active and counter electrodes, the circuitry generates a current stimulus of selected wave form, amplitude, duration, polarity. In some embodiments, the circuitry causes an electrical repulsion of active agents in order to deliver the active agent to the biological subject.

In some embodiments, circuitry includes, among other things, one or more computing devices such as a processor (e.g., a microprocessor, a quantum processor, qubit processor, etc.), a central processing unit (CPU), a digital signal processor (DSP), an application-specific integrated circuit (ASIC), a field programmable gate array (FPGA), or the like, or any combinations thereof, and can include discrete digital or analog circuit elements or electronics, or combinations thereof. In some embodiments, a module includes one or more ASICs having a plurality of predefined logic components. In some embodiments, a module includes one or more FPGAs, each having a plurality of programmable logic components.

In some embodiments, circuitry includes one or more electric circuits, printed circuits, electrical conductors, electrodes, electrocautery electrodes, cavity resonators, conducting traces, ceramic patterned electrodes, electro-mechanical components, transducers, and the like.

In some embodiments, circuitry includes one or more components operably coupled (e.g., communicatively, electromagnetically, magnetically, ultrasonically, optically, inductively, electrically, capacitively coupled, wirelessly coupled, or the like) to each other. In some embodiments, circuitry includes one or more remotely located components. In some embodiments, remotely located components are operably coupled, for example, via wireless communication. In some embodiments, remotely located components are operably coupled, for example, via one or more communication modules, receivers, transmitters, transceivers, or the like.

In some embodiments, circuitry includes memory that, for example, stores instructions or information. Non-limiting examples of memory include volatile memory (e.g., Random Access Memory (RAM), Dynamic Random Access Memory (DRAM), or the like), non-volatile memory (e.g., Read-Only Memory (ROM), Electrically Erasable Programmable Read-Only Memory (EEPROM), Compact Disc Read-Only Memory (CD-ROM), or the like), persistent memory, or the like. Further non-limiting examples of memory include Erasable Programmable Read-Only Memory (EPROM), flash memory, or the like. In some embodiments, memory is coupled to, for example, one or more computing devices by one or more instructions, information, or power buses.

In some embodiments, circuitry includes one or more computer-readable media drives, interface sockets, Universal Serial Bus (USB) ports, memory card slots, or the like, and one or more input/output components such as, for example, a graphical user interface, a display, a keyboard, a keypad, a trackball, a joystick, a touch-screen, a mouse, a switch, a dial, or the like, and any other peripheral device. In some embodiments, a module includes one or more user input/output components that are operably coupled to at least one computing device configured to control (electrical, electromechanical, software-implemented, firmware-implemented, or other control, or combinations thereof) at least one parameter associated with, for example, determining one or more tissue thermal properties responsive to detected shifts in turn-ON voltage.

In some embodiments, circuitry includes a computer-readable media drive or memory slot that is configured to accept signal-bearing medium (e.g., computer-readable memory media, computer-readable recording media, or the like). In some embodiments, a program for causing a system to execute any of the disclosed methods can be stored on, for example, a computer-readable recording medium, a signal-bearing medium, or the like. Non-limiting examples of signal-bearing media include a recordable type medium such as a magnetic tape, floppy disk, a hard disk drive, a Compact Disc (CD), a Digital Video Disk (DVD), Blu-Ray Disc, a digital tape, a computer memory, or the like, as well as transmission type medium such as a digital or an analog communication medium (e.g., a fiber optic cable, a waveguide, a wired communications link, a wireless communication link (e.g., receiver, transmitter, transceiver, transmission logic, reception logic, etc.). Further, non-limiting examples of signal-bearing media include, but are not limited to, DVD-ROM, DVD-RAM, DVD+RW, DVD-RW, DVD-R, DVD+R, CD-ROM, Super Audio CD, CD-R, CD+R, CD+RW, CD-RW, Video Compact Discs, Super Video Discs, flash memory, magnetic tape, magneto-optic disk, MINIDISC, non-volatile memory card, EEPROM, optical disk, optical storage, RAM, ROM, system memory, web server, or the like.

In some embodiments, circuitry includes acoustic transducers, electroacoustic transducers, electrochemical transducers, electromagnetic transducers, electromechanical transducers, electrostatic transducers, photoelectric transducers, radioacoustic transducers, thermoelectric transducers, ultrasonic transducers, and the like.

In some embodiments, circuitry includes electrical circuitry operably coupled with a transducer (e.g., an actuator, a motor, a piezoelectric crystal, a Micro Electro Mechanical System (MEMS), etc.). In some embodiments, circuitry includes electrical circuitry having at least one discrete electrical circuit, electrical circuitry having at least one integrated circuit, or electrical circuitry having at least one application specific integrated circuit. In some embodiments, circuitry includes electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of memory (e.g., random access, flash, read only, etc.)), electrical circuitry forming a communications device (e.g., a modem, communications switch, optical-electrical equipment, etc.), and/or any non-electrical analog thereto, such as optical or other analogs.

In some embodiments, circuitry includes one or more sensors configured to detect at least one physiological characteristic associated with a biological subject.

Having described the iontophoresis device and the circuitry, in some embodiments, an iontophoresis device comprises an electrode assembly including at least one electrode and a cosmetic composition; and circuitry is operably coupled to the electrode assembly and configured to concurrently generate at least a continuous direct current stimulus and a pulsed current stimulus to the same electrode, the continuous direct current stimulus and the pulsed current stimulus of a character and for a duration sufficient to deliver a cosmetic composition to a biological subject.

In some embodiments, during operation, the iontophoresis device includes circuitry configured to generate a continuous direct current stimulus having an average current density ranging from 0.01 mA/cm² to 0.5 mA/cm².

In some embodiments, the iontophoresis device includes circuitry configured to generate a continuous direct current stimulus having an average current density of 0.2 mA/cm².

In some embodiments, the iontophoresis device includes circuitry configured to generate a pulsed current stimulus having an average current density ranging from 0.01 mA/cm² to 10 mA/cm², a pulse width ranging from 50 microseconds to 1 milliseconds, and a pulse frequency ranging from 10 Hertz to 500 Hertz, and the duty cycle of pulses ranging from 1% to 90%.

In some embodiments, the iontophoresis device includes circuitry configured to generate a pulsed current stimulus having an average current density ranging from 0.01 mA/cm² to 10 mA/cm², a pulse width ranging from 50 microseconds to 1 milliseconds, at least one wave packet (or wave train) having from 2 to 20 pulses; a frequency of the wave packet ranging from 10 Hertz to 500 Hertz, and a duty cycle of pulses ranging from 1% to 90%.

In some embodiments, the iontophoresis device includes circuitry configured to generate a pulsed current stimulus having an average current density ranging from 0.01 mA/cm² to 10 mA/cm², a pulse width ranging from 50 microseconds to 1 milliseconds, at least one wave packet (or wave train) ranging from 2 to 20 pulses with alternating polarity, a frequency of the wave packet ranging from 10 Hertz to 500 Hertz, and a duty cycle of pulses ranging from 1% to 90%.

In some embodiments, the iontophoresis device includes circuitry configured to generate a pulsed current stimulus having an average current density of 0.2 mA/cm², an alternating pulse duration of 500 microseconds, and a pulse frequency of 200 Hertz.

In some embodiments of the iontophoresis device, the electrode assembly includes at least one reservoir holding an aqueous active agent composition.

In some embodiments of the iontophoresis device, the electrode assembly includes at least one active electrode electrically coupled to a reservoir holding a cosmetic composition, particularly an active agent aqueous composition; the electrode assembly operable to transdermally deliver the aqueous active agent composition to a biological subject responsive to one or more inputs from the circuitry configured to concurrently generate the continuous direct current stimulus and the pulsed current stimulus.

In some embodiments of the iontophoresis device, the electrode assembly is electrically coupled to at least one power source.

In some embodiments of the iontophoresis device, the electrode assembly includes at least one active electrode assembly and at least one counter electrode assembly.

In some embodiments of the iontophoresis device, the electrode assembly includes at least one reservoir holding a cosmetic composition comprising a face care or body care composition, comprising, in particular, an active agent chosen from humectant or moisturizing active agents, anti-ageing active agents, for example depigmenting active agents, active agents that act on cutaneous microcirculation, or seboregulating active agents, or a composition for making up the face or body, or a hair composition, in particular, a composition for washing the hair, for hair care or conditioning, for temporary form retention or shaping of the hair, for the temporary, semi-permanent or permanent dyeing of the hair, or for relaxing or permanent waving, in particular, a composition for relaxing, dyeing or bleaching the roots and hair, or a composition for the scalp, in particular, an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

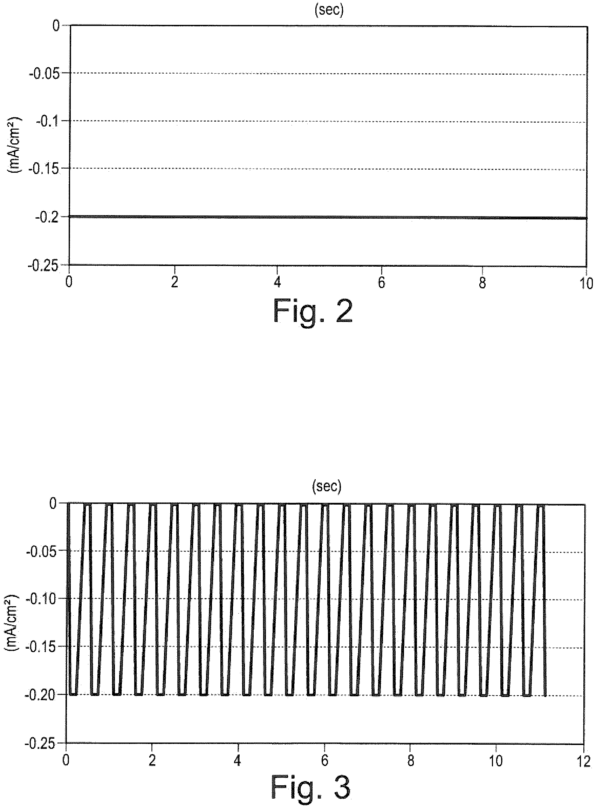

FIGURES 2-4 illustrate embodiments of electrical stimuli of representative current density waveforms generated by the circuitry of the iontophoresis device to administer vitamin C or any other aqueous active agent composition.

Referring to FIGURE 2, a first current density waveform is illustrated for iontophoresis. FIGURE 2 illustrates one embodiment of a waveform that can be generated by the circuitry and iontophoresis device of FIGURE 1. FIGURE 1 shows a continuous direct current waveform stimulus. The current density waveform is controlled an average constant value for the duration or any part of the iontophoresis treatment. In some embodiments, a current density waveform controlled at a constant value is referred to as continuous direct current, and the terms "direct current," "DC current," "galvanic current," and "DC" are interchangeable. In some embodiments, a negative current density means that the polarity at the first electrode 114 is negative. The second electrode 116 has positive polarity when the first electrode has negative polarity. Following convention, this means that current flows from the second positive electrode 116 to first negative electrode 114. Conversely, when the first electrode 114 has positive polarity and the second electrode 116 has negative polarity, current flows from the first positive electrode 114 to the second negative electrode 116, and this will be represented on a graph by a positive value of current density. Conventionally, electrons will be defined to flow in the opposite direction to current, i.e., from negative polarity to positive polarity. Current density is defined as ampere units per area units (of the cross section of the active electrode).

While FIGURE 2 depicts a certain continuous direct current density value and duration, it should be appreciated that the illustrated values are exemplary. In some embodiments of the continuous direct current waveform of FIGURE 2, the average current density is controlled at or below 0.5 mA/cm². In some embodiments of the continuous direct current waveform of FIGURE 2, the average current density is controlled at or below 0.2 mA/cm². In some embodiments of the continuous direct current waveform of FIGURE 2, the average current density is controlled between 0.01 mA/cm² to 0.5 mA/cm². In some embodiments of the continuous direct current waveform of FIGURE 2, the average current density is controlled at any one of the following values, 0.01, 0.05, 0.1, 0.15, 0.2, 0.25, 0.3, 0.35, 0.4, 0.45, 0.5 mA/cm² or in a range between any two values serving as endpoints. In some embodiments of the continuous direct current waveform of FIGURE 2, the amplitude is controlled at any of the above values and electrical current is applied for a duration of at least 1 minute. In some embodiments of the continuous direct current waveform of FIGURE 2, the amplitude is controlled at any of the above values and electrical current is applied for a duration of from 10 to 20 minutes. In some embodiments of the continuous direct current waveform of FIGURE 2, the amplitude is controlled at any of the above values and electrical current is applied for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some of the embodiments of the continuous direct current waveform of FIGURE 2, the first electrode 114 is negative and the second electrode 116 is positive for duration of the waveform.

Referring to FIGURE 3, a second current density waveform is illustrated for iontophoresis. FIGURE 3 illustrates one embodiment of a waveform that can be generated by the circuitry and the iontophoresis device of FIGURE 1. FIGURE 3 shows a pulse wave. The current density waveform is controlled in negative pulses (polarity is negative at electrode 114) for the duration or any part of the iontophoresis treatment. In some embodiments, the polarity can be reversed. The pulses of FIGURE 3 are unipolar, meaning that current travels in one direction. A pulse of FIGURE 3 has a maximum amplitude. The pulse waveform will increase from a minimum amplitude, reach the maximum amplitude, and then decrease to the minimum amplitude, reside at the minimum amplitude, and the cycle will repeat. In some embodiments, a pulse is counted starting from the minimum amplitude, reaching the maximum amplitude, and then returning to the minimum amplitude. Thus, a pulse does not include the period of the minimum amplitude. In some embodiments, a pulse cycle does include the period at the minimum amplitude. In some embodiments, the durations of the maximum and minimum amplitudes are the same.

In some embodiments, the pulse wave is expressed to have a % duty cycle. In some embodiments, expressing a % duty cycle with respect to a pulse wave means that the electrical current is on for the % duty cycle. For example, 50 % duty cycle means electrical current is on for 50% and off for 50% of the pulse cycle, 30% duty cycle means electrical current is on for 30% and off for 70% of the pulse cycle. In some embodiments, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In some embodiments, the % duty cycle of a respective bipolar pulse is 0.01, 5, 10, 15, 20, 25, 30, 35, 40, 45, 50, 55, 60, 65, 70, 75, 80, 85, 90, 95, 100 or any range between any two values serving as endpoints. In some embodiments, the duty cycle of pulses ranges from 1% to 90%. In some embodiments, the pulse, meaning the "on" period can be expressed as a duration having units of time. In some embodiments, the pulse "off" period can be expressed as a duration. In some embodiments, the pulse wave will be expressed in hertz, meaning cycles per second. In some embodiments, the pulses can be reversed by alternating the polarities of the first and second electrodes between negative and positive. In some embodiments, bipolar pulses, alternating pulses, bidirectional pulses, and reverse pulses mean the same. In some embodiments, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in some embodiments, the duration of negative current density pulse does not have to be the same duration of a positive current density pulse. In some embodiments, the duration of pulses does not have to be the same duration, regardless whether the pulses are negative or positive.

In some embodiments, a pulse waveform can combine two or more pulse waveforms concurrently or alternatively. In some embodiments, a pulse waveform can include negative pulses, followed by positive pulses. Thus, having a maximum and minimum amplitude for the negative pulses and a maximum and a minimum amplitude for the positive pulses.

While FIGURE 3 depicts certain current density values of the maximum and minimum pulse amplitudes and pulse duration, it should be appreciated that the illustrated values are exemplary only. In some embodiments of the current waveform of FIGURE 3, the average current density is controlled in pulses and each pulse maximum is controlled at most at 0.2 mA/cm² and the minimum amplitude is 0. In some embodiments of the current waveform of FIGURE 3, the average current density is controlled in pulses and each pulse maximum is controlled at or below 0.5 mA/cm² and the minimum amplitude is 0. In some embodiments of the current waveform of FIGURE 3, the average current density is controlled in pulses and each pulse maximum is controlled from 0.2 mA/cm² to 0.5 mA/cm² and the minimum amplitude is 0. In some embodiments of the current waveform of FIGURE 3, the average current density is controlled in pulses and each pulse maximum is controlled from 0.01 mA/cm² to 10 mA/cm² and the minimum amplitude is 0. In some embodiments of the current waveform of FIGURE 3, the average current density is controlled in pulses and each pulse maximum is controlled from 0.05 mA/cm² to 0.5 mA/cm² and the minimum amplitude is 0. In some embodiments of the current waveform of FIGURE 3, the average current density is controlled in pulses and each pulse maximum is controlled at or below 0.2 mA/cm² and the minimum amplitude is 0. In some embodiments of the current waveform of FIGURE 3, the average current density is controlled in pulses and each pulse maximum is controlled at 0.01, 0.05, 0.1, 0.15, 0.2, 0.25, 0.3, 0.35,0.4, 0.45, 0.5 mA/cm² or in the range between any two values serving as endpoints. In some embodiments, the current density is given as the root-mean-square (rms). In some embodiments, the current density is given as the average current density. In some embodiments, the current density can be given as the peak current density, which can be as high as 1 mA/cm² or 2mA/ cm² with a duty cycle of 50% and 25%, respectively.

In some embodiments, the pulse has a positive constant slope (other than vertical) to the maximum amplitude, followed by a duration at the constant maximum amplitude, followed by a negative constant slope (other than vertical) to 0, followed by a duration at 0. In some embodiments, the minimum can be other than 0. In some embodiments, the slope can be other than constant, such as exponential. In some embodiments of the current waveform of FIGURE 3, the pulses are not triangular. In some embodiments of FIGURE 3, the pulses are a square wave, wherein the maximum and the minimum amplitudes are of the same duration or not. In some embodiments of the current waveform of FIGURE 3, the pulses are not square wave. In some embodiments, the pulse wave is sinusoidal, non-sinusoidal, or any combination. In some embodiments, the pulse wave is periodic square wave, rectangular wave, saw tooth wave, spiked wave, trapezoidal wave, triangle wave, or combinations thereof.

In some embodiments of FIGURE 3, the duration of the maximum amplitude of the pulses is less than the duration of the minimum amplitude between pulses. In some embodiments of FIGURE 3, the pulse duration (or width) is defined as the time between the minimums with a maximum (either positive or negative) between the two minimums. In some embodiments, the pulse duration (or width) is given in units of time. In some embodiments, the pulse duration (or width) ranges from 50 microseconds to 1 milliseconds. In some embodiments, the pulse duration (or width) ranges from 200 microseconds to 300 microseconds. In some embodiments, the pulse duration (or width) ranges from 10 microseconds to 500 microseconds. In some embodiments, the pulse duration (or width) ranges from 50 microseconds to 5 milliseconds. In some embodiments, the pulse duration (or width) is less than 50 microseconds or greater than 5 milliseconds. In some embodiments, the pulse duration (or width) is 500 microseconds. In some embodiments of FIGURE 3, the duration of the maximum amplitude of the pulses is greater than the duration of the minimum amplitude between pulses. In some embodiments of the current waveform of FIGURE 3, the minimum amplitude is 0 mA/cm². In some embodiments of FIGURE 3, the minimum amplitude is greater than 0 mA/cm² (meaning "more" negative than 0 with respect to FIGURE 3). In some embodiments of the current waveform of FIGURE 3, the maximum (and minimum) amplitude can increase from pulse to pulse. In some embodiments of the current waveform of FIGURE 3, the maximum (and minimum) amplitude can decrease from pulse to pulse. In some embodiments of the current waveform of FIGURE 3, the maximum (and minimum) amplitude can increase from pulse to pulse, and then decrease from pulse to pulse, and repeat.

In some embodiments of the current waveform of FIGURE 3, the average current density is controlled in pulses at any of the above values and the rate of pulses is from 100 hertz to 300 hertz. In some embodiments of the current waveform of FIGURE 3, the average current density is controlled in pulses at any of the above values and the rate of pulses is from 1 hertz to 200 hertz. In some embodiments, the pulse is less than 1 hertz. In some embodiments of the current waveform of FIGURE 3, the average current density is controlled in pulses at any of the above values and the rate of pulses is from 1 hertz to 500 hertz. In some embodiments of the current waveform of FIGURE 3, the average current density is controlled in pulses at any of the above values and the rate of pulses is 200 hertz. In some embodiments of the current waveform of FIGURE 3, the average current density is controlled in pulses at any of the above values and the rate of pulses is from 10 hertz to 500 hertz. In some embodiments of the current waveform of FIGURE 3, the average current density is controlled in pulses wherein the rate of pulses is from 1 hertz to 500 hertz, or any value in between in increments of 1 hertz.

In some of the embodiments of FIGURE 3, a pulse wave travels in wave packets (or wave trains). In some embodiments, the wave packets are defined by the number of pulses, the duration or width of pulses in the packet, the average current density of pulses, the duty cycle of pulses in the wave packet, and the frequency of the wave packets. In some embodiments, the wave packet can have from 2 pulses or greater with or without alternating polarity. In some embodiments of FIGURE 3, the wave packet can have from 2 pulses to 20 pulses with or without alternating polarity. In some embodiments of FIGURE 3, the wave packets can be generated at a frequency from 10 Hertz or greater. In some embodiments of FIGURE 3, the wave packets can be generated at a frequency from 10 Hertz to 500 Hertz. In some embodiments, the duty cycle of the pulses in the wave packets ranges from 1% to 90%. In some embodiments, the pulses have an average current density of 0.01mA/cm² to 10 mA/cm².

In some embodiments of the current waveform of FIGURE 3, the iontophoresis treatment is applied for a duration of from 30 seconds to 5 minutes. In some embodiments of the current waveform of FIGURE 3, the iontophoresis treatment is applied for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some embodiments of the current waveform of FIGURE 3, the first electrode 114 has negative polarity and the second electrode 116 has positive polarity when the pulses extend below 0. However, the first electrode 114 is positive and the second electrode 116 is negative when the pulses extend above 0. Thus, indicating a reversal in the direction of current.

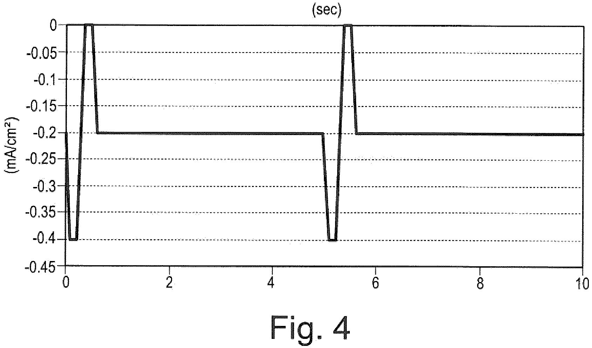

Referring to FIGURE 4, a third current density waveform is illustrated for iontophoresis. FIGURE 4 illustrates one embodiment of a waveform that can be generated by the iontophoresis device of FIGURE 1. FIGURE 4 shows a continuous direct current concurrent with a pulse wave. The current density waveform is controlled in negative unipolar pulses and between pulses, the current density is controlled as continuous direct current. Stated another way, the current waveform of FIGURE 4 can be described as the combination between a direct current and a bidirectional pulse taken as an offset direct current with a duty cycle smaller than 100%. The two waveforms are applied concurrently for the duration or any part of the iontophoresis treatment. Specifically, the pulse wave has an on and off period. The superposition of the pulse wave on top of continuous direct current causes the current profile to show the pulse beginning at the constant value of the direct current. The pulse reaches a maximum for the predetermined duration and then the profile goes to 0. After the off period from the 0 value, the continuous direct current is applied until then next pulse. Thus, the current density of the waveform can be described as the addition of continuous direct current of a first amplitude with a pulse of a second amplitude, wherein the pulse has an off period before applying the direct current again. A pulse is counted starting from the direct current amplitude, reaching the maximum pulse amplitude, and then returning to the minimum amplitude or 0. Thus, a pulse does not include the period of the minimum amplitude at 0. A pulse cycle does include the period at the minimum amplitude. In some embodiments, the durations of the maximum and minimum amplitudes are the same.

In some embodiments, the pulse is expressed to have a % duty cycle. In some embodiments, expressing a % duty cycle with respect to a pulse wave means that the electrical current is on for the % duty cycle. For example, 50 % duty cycle of pulses means electrical current is on for 50% and off for 50% of the pulse cycle, 30% duty cycle of pulses means electrical current is on for 30% and off for 70% of the pulse cycle. In some embodiments, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In some embodiments, the % duty cycle of a respective bipolar pulse is 0.01, 5, 10, 15, 20, 25, 30, 35, 40, 45, 50, 55, 60, 65, 70, 75, 80, 85, 90, 95, 100 or any range between any two values serving as endpoints. In some embodiments, the duty cycle of pulses ranges from 1% to 90%. In some embodiments, the pulse, meaning the "on" period can be expressed as a duration. In some embodiments, the pulse "off" period can be expressed as a duration. In some embodiments, the pulse wave is expressed in hertz, meaning cycles per second. In some embodiments, the pulses can be reversed by alternating the polarities of the first and second electrodes between negative and positive. In some embodiments, bipolar pulses, alternating pulses, bidirectional pulses, and reverse pulses mean the same. In some embodiments, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in some embodiments, the duration of negative current density pulse does not have to be the same duration of a positive current density pulse. In some embodiments, the duration of pulses does not have to be the same duration, regardless whether the pulses are negative or positive.

In some embodiments, a pulse waveform can combine two or more pulse waveforms concurrently or alternatively. In some embodiments, a pulse waveform can include negative pulses, followed by positive pulses. Thus, having a maximum and minimum amplitude for the negative pulses and a maximum and a minimum amplitude for the positive pulses.

While FIGURE 4 depicts certain current density values of the maximum and minimum pulse amplitudes and pulse duration, it should be appreciated that the illustrated values are exemplary only. In some embodiments of the current waveform of FIGURE 4, concurrent with a direct current, the average current density is controlled in pulses and each pulse maximum is controlled at most at 0.2 mA/cm² and the minimum amplitude is 0. This means that the addition of the pulse to the direct current total is 0.4 mA/cm². In some embodiments of the current waveform of FIGURE 4, concurrent with a direct current, the average current density is controlled in pulses and each pulse maximum is at or below 0.5 mA/cm² and the minimum amplitude is 0, and direct current average current density is controlled at or below an average of 0.5 mA/cm². In some embodiments of the current waveform of FIGURE 4, concurrent with a direct current, the average current density is controlled in pulses and each pulse maximum is controlled from 0.2 mA/cm² to 0.5 mA/cm² and the minimum amplitude is 0, and the direct current average current density is controlled from 0.2 mA/cm² to 0.5 mA/cm². In some embodiments of the current waveform of FIGURE 4, concurrent with a direct current, the average current density is controlled in pulses and each pulse maximum is controlled at or below 0.2 mA/cm² and the minimum amplitude is 0, and the direct current average current density is controlled at or below 0.2 mA/cm². In some embodiments of the current waveform of FIGURE 4, concurrent with direct current, the average current density is controlled in pulses and each pulse maximum is controlled from 0.01 mA/cm² to 10 mA/cm², and the direct current average current density is controlled from 0.01 mA/cm² to 0.5 mA/cm², In some embodiments of the current waveform of FIGURE 4, concurrent with direct current, the average current density is controlled in pulses and the direct current and each pulse maximum is controlled on average at 0.01, 0.05, 0.1, 0.15, 0.2, 0.25, 0.3, 0.35,0.4, 0.45, 0.5 mA/cm² or in the range between any two values serving as endpoints. In some embodiments, the current density is given as the root-mean-square (rms). In some embodiments, the current density is given as the average current density. In some embodiments, the current density can be given as the peak current density, which can be as high as 1 mA/cm² or 2mA/ cm² with a duty cycle of 50% and 25%, respectively.

In some embodiments of FIGURE 4, the pulses are triangular with a defined maximum and minimum, wherein the maximum and the minimum amplitudes are of the same duration. Specifically, the pulse has a positive constant slope (other than vertical) to the maximum amplitude, followed by a period at the constant maximum amplitude, followed by a negative constant slope (other than vertical) to 0, followed by a period at 0. In some embodiments, the minimum can be other than 0. In some embodiments, the slope can be other than constant, such as exponential. In some embodiments, the pulse wave is sinusoidal, non-sinusoidal, or any combination. In some embodiments, the pulse wave is periodic square wave, rectangular wave, saw tooth wave, spiked wave, trapezoidal wave, triangle wave, or combinations thereof.

In some embodiments of FIGURE 4, the duration of the maximum amplitude of the pulses is less than the duration of the minimum amplitude between pulses. In some embodiments of FIGURE 4, the pulse duration (or width) is defined as the time between the minimums with a maximum (either positive or negative) between the two minimums. In some embodiments, the pulse duration (or width) is given in units of time. In some embodiments, the pulse duration (or width) ranges from 50 microseconds to 1 milliseconds. In some embodiments, the pulse duration (or width) ranges from 200 microseconds to 300 microseconds. In some embodiments, the pulse duration (or width) ranges from 10 microseconds to 500 microseconds. In some embodiments, the pulse duration (or width) ranges from 50 microseconds to 5 milliseconds. In some embodiments, the pulse duration (or width) is less than 50 microseconds or greater than 5 milliseconds. In some embodiments, the pulse duration (or width) is 500 microseconds. In some embodiments of FIGURE 4, the duration of the maximum amplitude of the pulses is greater than the duration of the minimum amplitude between pulses. In some embodiments of the current waveform of FIGURE 4, the minimum amplitude is 0 mA/cm². In some embodiments of FIGURE 4, the minimum amplitude is greater than 0 mA/cm² (meaning "more" negative than 0 with respect to FIGURE 4). In some embodiments of the current waveform of FIGURE 3, the maximum (and minimum) amplitude can increase from pulse to pulse. In some embodiments of the current waveform of FIGURE 4, the maximum (and minimum) amplitude can decrease from pulse to pulse. In some embodiments of the current waveform of FIGURE 4, the maximum (and minimum) amplitude can increase from pulse to pulse, and then decrease from pulse to pulse, and repeat.

In some embodiments of the current waveform of FIGURE 4, the average current density is controlled as direct current concurrently with pulses at any of the above values and the rate of pulses is from 100 hertz to 300 hertz. In some embodiments of the current waveform of FIGURE 4, the current density is controlled as direct current concurrently with pulses at any of the above values and the rate of pulses is from 1 hertz to 500 hertz. In some embodiments of the current waveform of FIGURE 4, the average current density is controlled as direct current concurrently with pulses wherein the rate of pulses is from 1 hertz to 500 hertz, or any value in between in increments of 1 hertz. In some embodiments of the current waveform of FIGURE 4, concurrent with direct current, the average current density is controlled in pulses at any of the above values and the rate of pulses is from 10 hertz to 500 hertz. In some embodiments of the current waveform of FIGURE 4, each pulse has a duration of between 0.001 seconds to 1 second or any value in between. In some embodiments of the current waveform of FIGURE 4, concurrent with direct current, the average current density is controlled in pulses and the rate of pulses is 200 hertz and each pulse has a duration of 500 microseconds.

In some of the embodiments of FIGURE 4, the pulses are applied as a wave packet (or wave train). In some embodiments, the wave packets are defined by the number of pulses, the duration or width of pulses in the packet, the average current density of pulses, the duty cycle of pulses in the wave packet, and the frequency of the wave packets. In some embodiments, the wave packet can have from 2 pulses or greater with or without alternating polarity. In some embodiments of FIGURE 4, the wave packet can have from 2 pulses to 20 pulses with or without alternating polarity. In some embodiments of FIGURE 4, the wave packets can be generated at a frequency from 10 Hertz or greater. In some embodiments of FIGURE 4, the wave packets can be generated at a frequency from 10 Hertz to 500 Hertz. In some embodiments, the duty cycle of the pulses in the wave packets ranges from 1% to 90%. In some embodiments, the pulses of wave packets have an average current density of 0.01mA/cm² to 10 mA/cm².

In some embodiments of the current waveform of FIGURE 4, the iontophoresis treatment is applied for a duration (in minutes) of 1, 1.5, 2, 2.5, 3, 3.5, 4, 4.5, 5 or any range between any two values serving as endpoints. In some embodiments of the waveform of FIGURE 4, the electrical current is applied as continuous direct current and in pulses for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some embodiments of the current waveform of FIGURE 4, the pulses are unipolar pulses. In some embodiments of the current waveform of FIGURE 4, the first electrode 114 has negative polarity and the second electrode 116 has positive polarity when the pulses extend below 0. However, the first electrode 114 is positive and the second electrode 116 is negative when the pulses extend above 0. Thus, indicating a reversal in the direction of current.

In some embodiments, the current waveforms of FIGURES 2, 3 and 4 can be combined to provide concurrent waveforms for the administration of aqueous active agent compositions, such as vitamin C compositions, into the skin via the use of iontophoresis. In some embodiments, the current waveforms of FIGURES 2, 3 and 4 can be combined to provide concurrent waveforms for the administration of one or more of a face care or body care composition, comprising, in particular, an active agent chosen from humectant or moisturizing active agents, anti-ageing active agents, for example depigmenting active agents, active agents that act on cutaneous microcirculation, or seboregulating active agents, a composition for making up the face or body, a hair composition, in particular, a composition for washing the hair, for hair care or conditioning, for temporary form retention or shaping of the hair, for the temporary, semi-permanent or permanent dyeing of the hair, or for relaxing or permanent waving, in particular, a composition for relaxing, dyeing or bleaching the roots and hair, and a composition for the scalp, in particular, an antidandruff composition, a composition for preventing hair loss or for promoting regrowth of the hair, an anti- seborrheic composition, an anti-inflammatory composition, an anti-irritation or soothing composition, a mark-preventing composition or a composition for stimulating or protecting the scalp.

In some embodiments, the current waveforms of FIGURES 2, 3 and 4 can be combined to provide combinations of waveforms that are generated by the circuitry of the iontophoresis device of FIGURE 1. In some embodiments, the current waveform of any FIGURE 2-4 is applied for a first duration, followed by a different current waveform of any FIGURE 2-4 for a second duration or vice versa. In some embodiments two or more different current waveforms can be cycled for the entire electrical current treatment duration. The particulars of the waveform as described above for FIGURES 2-4 are similarly applicable to combined treatments applying the two or more different waveforms. That is, any one or more of the embodiments of the direct current waveform can be combined with any one or more of the pulse waveforms in sequence or simultaneously.

In the case of the waveforms of FIGURES 2, 3, and 4, the peak voltage at maximum peak is 99 volts. In some embodiments, the maximum duration of conducting electrical current is 120 minutes during the iontophoresis treatment.

Every embodiment of the current density waveforms described in connection with FIGURES 2, 3 and 4 and the combination of waveforms can be used for iontophoresis of every embodiment of the compositions.

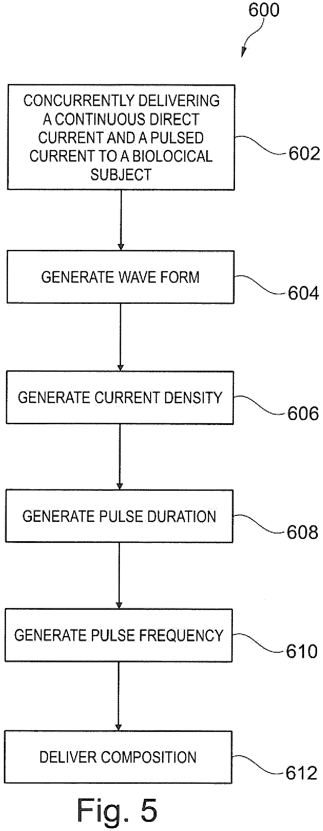

FIGURE 5 illustrates embodiments of a method 600 for delivering a cosmetic composition through the generation of electrical stimuli of certain waveform types. In some embodiments, the method includes a step 602 for concurrently delivering a continuous direct current and a pulsed current to a biological subject, the continuous direct current and the pulsed current is of a character and for a duration sufficient to deliver a cosmetic composition to the biological subject.

In some embodiments, the illustrated steps 604, 606, 608, and 610 are optional. Further, in some embodiments, the sequence of the steps 604, 606, 608, and 610 can be in any order and is not confined to the illustration. In some embodiments, the method 600 includes a step 604 for generating waveforms. In some embodiments, the user makes selections that cause the iontophoresis device to generate the selected waveform or waveforms that constitute the electrical stimuli. In some embodiments, the method 600 includes a step 606 for generating current density. In some embodiments, the user makes selections that cause the iontophoresis device to generate the selected current density. In some embodiments, the method 600 includes a step 608 for generating pulse duration. In some embodiments, the user makes selections that cause the iontophoresis device to generate the selected pulse duration. In some embodiments, the method 600 includes a step 610 for generating pulse frequency. In some embodiments, the user makes selections that cause the iontophoresis device to generate the selected pulse frequency.

In some embodiments, the method 600 for concurrently delivering the continuous direct current and the pulsed current to a biological subject includes generating a continuous direct current stimulus having an average current density ranging from 0.01 mA/cm² to 0.5 mA/cm².

In some embodiments, the method 600 for concurrently delivering the continuous direct current and the pulsed current to a biological subject includes generating a continuous direct current stimulus having an average current density of 0.2 mA/cm².

In some embodiments, the method 600 for concurrently delivering the continuous direct current and the pulsed current to a biological subject includes generating a pulsed current stimulus having an average current density ranging from 0.01 mA/cm² to 10 mA/cm², a pulse duration ranging from 50 microseconds to 1 milliseconds, and a pulse frequency ranging from 10 Hertz to 500 Hertz, and a duty cycle of pulses ranging from 1% to 90%.

In some embodiments, the method 600 for concurrently delivering the continuous direct current and the pulsed current to a biological subject includes generating a pulsed alternating current stimulus having an average current density of 0.2 mA/cm², a pulse duration of 500 microseconds, and a pulse frequency of 200 Hertz.

In some embodiments, the method 600 for concurrently delivering the continuous direct current and the pulsed current to a biological subject includes generating a pulsed current having an average current density ranging from 0.01 mA/cm² to 10 mA/cm², a pulse width ranging from 50 microseconds to 1 milliseconds, at least one wave packet (or wave train) ranging from 2 to 20 pulses, a frequency of wave packets ranging from 10 Hertz to 500 Hertz, and a duty of pulses ranging from 1% to 90%.

In some embodiments, the method 600 for concurrently delivering the continuous direct current and the pulsed current to a biological subject includes generating a pulsed current stimulus having an average current density ranging from 0.01 mA/cm² to 10 mA/cm², a pulse width ranging from 50 microseconds to 1 milliseconds, at least one wave packet (wave train) having from 2 to 20 pulses with alternating polarity, a frequency of wave packets ranging from 10 Hertz to 500 Hertz, and a duty cycle of pulses ranging from 1% to 90%.

In some embodiments, the method 600 for concurrently delivering the continuous direct current and the pulsed current to a biological subject includes generating a pulsed current having sinusoidal waveforms, non-sinusoidal waveforms, or combinations thereof.

In some embodiments, the method 600 for concurrently delivering the continuous direct current and the pulsed current to a biological subject includes generating a pulsed current having periodic square waveforms, rectangular waveforms, saw tooth waveforms, spiked waveforms, trapezoidal waveforms, triangle waveforms, or combinations thereof.

In some embodiments, the method 600 comprises delivering a cosmetic composition chosen from a face care or body care composition, comprising in particular, an active agent chosen from humectant or moisturizing active agents, anti-ageing active agents, for example depigmenting active agents, active agents that act on cutaneous microcirculation, or seboregulating active agents, or a composition for making up the face or body.

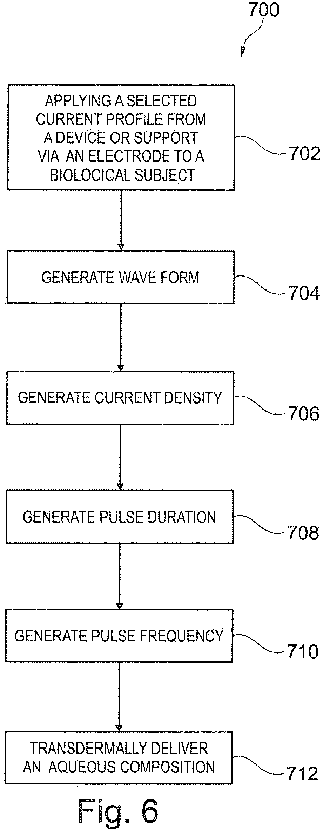

FIGURE 6 illustrates embodiments of a method 700 for delivering an aqueous active agent composition, such as an aqueous vitamin C composition through the skin to a biological subject through the generation of electrical stimuli of certain waveform types.

In some embodiments, the method 700 includes step 702 for applying a selected current profile, either continuous direct current, pulsed current or a combination of both, from any device and/or support comprising at least one electrode to a biological subject, the continuous direct current, the pulsed current or the combination of both is of a character and for a duration sufficient to transdermally deliver an aqueous composition to a biological subject, thus, transporting different rates of vitamin C across the skin in accordance to the selected current mode.

In some embodiments, the illustrated steps 704, 706, 708, and 710 are optional. Further, in some embodiments, the sequence of the steps 704, 706, 708, and 710 can be in any order and is not confined to the illustration. In some embodiments, the method 700 includes a step 704 for generating waveforms. In some embodiments, the user makes selections that cause the iontophoresis device to generate the selected waveform or waveforms that constitute the electrical stimuli. In some embodiments, the method 700 includes a step 706 for generating current density. In some embodiments, the user makes selections that cause the iontophoresis device to generate the selected current density. In some embodiments, the method 700 includes a step 708 for generating pulse duration. In some embodiments, the user makes selections that cause the iontophoresis device to generate the selected pulse duration. In some embodiments, the method 700 includes a step 710 for generating pulse frequency. In some embodiments, the user makes selections that cause the iontophoresis device to generate the selected pulse frequency. In step 712, the method 700 includes transdermally delivering an aqueous composition.

In some embodiments of the method 700 of delivering an aqueous vitamin C composition through the skin, applying a selected current profile to a biological subject includes generating a continuous direct current stimulus having an average current density ranging from 0.01 mA/cm² to 0.5 mA/cm².

In some embodiments of the method 700 of delivering an aqueous vitamin C composition through the skin, applying a selected current profile to a biological subject includes generating a continuous direct current stimulus having an average current density of 0.2 mA/cm².

In some embodiments of the method 700 of delivering an aqueous vitamin C composition through the skin, applying a selected current profile to a biological subject includes generating a pulsed current having sinusoidal waveforms, non-sinusoidal waveforms, or combinations thereof.

In some embodiments of the method 700 of delivering an aqueous vitamin C composition through the skin, applying a selected current profile to a biological subject includes generating a pulsed current having periodic square waveforms, rectangular waveforms, saw tooth waveforms, spiked waveforms, trapezoidal waveforms, triangle waveforms, or combinations thereof.

In some embodiments of the method 700 of delivering an aqueous vitamin C composition through the skin, applying a selected current profile to a biological subject includes concurrently delivering the continuous direct current and the pulsed current and generating a pulsed current stimulus having an average current density ranging from 0.05 mA/cm² to 0.5 mA/cm²; a pulse duration ranging from 200 microseconds to 300 microseconds; and a pulse frequency ranging from 100 Hertz to 300 Hertz.

In some embodiments, the method 700 of delivering an aqueous vitamin C composition through the skin further comprises transdermally delivering an aqueous composition including, one or more of vitamin C, vitamin C derivatives, ions of vitamin C, and ions of vitamin C derivatives, and at least an anionic or non-ionic polymer.

In some embodiments, the method 700 of delivering an aqueous vitamin C composition through the skin further comprises transdermally delivering an aqueous composition including, one or more of vitamin C, vitamin C derivatives, ions of vitamin C, and ions of vitamin C derivatives present in amounts ranging from 0.1 % to 20 % by weight; and at least an anionic or non-ionic polymer ranging from 0.01 % to 10% by weight; and water present in an amount of at least 30 % by weight.

In some embodiments, the method 700 of delivering an aqueous vitamin C composition through the skin, further comprises transdermally delivering an aqueous composition including, one or more of vitamin C, vitamin C derivatives, ions of vitamin C, and ions of vitamin C derivatives present in amounts ranging from 0.01 % to 30 % by weight; one or more anionic polymers present in amounts ranging from 0.01 % to 20 % by weight; and water present in an amount of at least 30 % by weight.

In some embodiments, the method 700 of delivering an aqueous vitamin C composition through the skin, further comprises transdermally delivering an aqueous composition including, one or more of vitamin C, vitamin C derivatives, ions of vitamin C, and ions of vitamin C derivatives present in amounts ranging from 0.01 % to 30 % by weight; one or more non-ionic polymers present in amounts ranging from 0.01 % to 20 % by weight; and water present in an amount of at least 30 % by weight.

In some embodiments of the method 700 of delivering an aqueous vitamin C composition through the skin, transdermally delivering the aqueous active agent composition includes generating a continuous direct current stimulus having an average current density ranging from 0.01 mA/cm² to 0.5 mA/cm²; and generating a pulsed current stimulus having an average current density ranging from 0.01 mA/cm² to 10 mA/cm²; a pulse duration ranging from 10 microseconds to 500 microseconds; and a pulse frequency ranging from 10 Hertz to 500 Hertz; the continuous direct current and the pulsed current of a duration sufficient to transdermally deliver an aqueous active agent composition to a biological subject.

An iontophoresis composition for use with the iontophoresis methods described above in relation to FIGURES 5 and 6 is disclosed.

In some embodiments, the iontophoresis composition includes one or more of vitamin C, vitamin C derivatives, ions of vitamin C, and ions of vitamin C derivatives present in amounts ranging from 0.1 % to 30 % by weight; and water present in an amount of at least 20 % by weight; the iontophoresis composition having an aqueous phase that is at least 30% by weight relative to the total weight of the iontophoresis composition.

In some embodiments, the iontophoresis composition further comprises one or more ionic polymers present in amounts ranging from 0.01 % to 10% by weight; wherein the one or more of vitamin C, vitamin C derivatives, ions of vitamin C, and ions of vitamin C derivatives are present in amounts ranging from 0.1 % to 30 % by weight.

In some embodiments, the iontophoresis composition further comprises one or more non-ionic polymers present in amounts ranging from 0.01 % to 20% by weight; wherein the one or more of vitamin C, vitamin C derivatives, ions of vitamin C, and ions of vitamin C derivatives are present in amounts ranging from 0.01 % to 30 % by weight.

In some embodiments, the iontophoresis composition further comprises a pH ranging from 2 to 7.5.

In some embodiments of the iontophoresis composition, the composition may comprise one or more silicon materials, which may include one or more silicon surface-active agents. In some embodiments of an iontophoresis composition, the silicon-containing surface active agents are selected from polydimethylsiloxane, poly[oxy(dimethylsilylane)], polyvinyl siloxane, cyclohexasiloxane, derivatives thereof, or any combination thereof.

In some embodiments of the iontophoresis composition, the anionic polymers and nonionic polymers are selected from ammonium polyacryloyldimethyl taurate, sodium carboxymethyl cellulose purified, sodium acryloyldimethyltaurate/VP crosspolymer and hydroxypropyl methyl cellulose (HPMC),
and from acrylonitrile/methyl methacrylate/vinylidene chloride copolymer, biosaccharide gum-1, sodium styrene/maleic anhydride copolymer, xanthan gum, ammonium polyacryloyldimethyl taurate, derivatives thereof, their ions, and any combination thereof.

In some embodiments of the iontophoresis composition, vitamin C derivatives are selected from ascorbyl palmitate and magnesium ascorbyl phosphate, ascorbyl tetra-isopalmitoyl, tetrahexyldecyl ascorbate, sodium ascorbyl phosphate, and any combination thereof.

In some embodiments of the iontophoresis composition, the composition further comprises a vitamin, a fat, a solvent, a humectant, a viscosity reducer, a preservative, a chelating agent, a viscosity controller, a skin conditioner, an emollient, an emulsifier, a cleansing agent, an emulsion stabilizer, a viscosity increaser, an antioxidant, a binder, a skin bleaching agent, a pH adjuster, a buffering agent, a denaturant, a bulking agent, an opacifying agent.

In some embodiments of the iontophoresis composition, the composition includes ionic polymers and nonionic polymers selected from biosaccharide gum-1 (and) sodium levulinate (and) glyceryl caprylate (and) sodium anisate, acrylates/c10-30 alkyl acrylate crosspolymer, carbomer, sodium styrene/maleic anhydride copolymer, nylon-12, xanthan gum, derivatives thereof, their ions, or any combination thereof.

In some embodiments, the iontophoresis composition has a pH greater than 4.5.

In some embodiments, the iontophoresis composition has a pH from 4.5 to 7.4.

In some embodiments, the iontophoresis composition has a pH from 4.5 to 6.3.

In some embodiments, the iontophoresis composition has a pH from 5.7 to 6.3.

In some embodiments, the iontophoresis composition includes one or more vitamins selected from vitamin B5, vitamin A, vitamin B3, and vitamin E.

In some embodiments, the iontophoresis composition includes one or more fats selected from nut oils, seed oils, and plant oils.

In some embodiments, the iontophoresis composition includes one or more solvents selected from water, deionized water, and Eau de la Roche-Posay™.

In some embodiments, the iontophoresis composition includes one or more humectants selected from glycerin, caprylyl glycol, and sodium hyaluronate.

In some embodiments, the iontophoresis composition includes one or more viscosity reducers selected from glycerine.

In some embodiments, the iontophoresis composition includes one or more preservatives selected from phenoxyethanol, salicylic acid, and sodium methylparaben.

In some embodiments, the iontophoresis composition includes one or more chelating agents selected from disodium EDTA.

In some embodiments, the iontophoresis composition includes one or more viscosity controllers selected from disodium EDTA, ammonium polyacryldimethyltauramide, and nylon-12.

In some embodiments, the iontophoresis composition includes one or more skin conditioners selected from C12-15 alkyl benzoate, caprylyl glycol, glyceryl stearate and polyethylene glycol 100 Stearate, tocopheryl acetate, sodium hyaluronate, ethylhexyl palmitate, dimethicone and dimethiconol, dimethicone, dimethicone and dimethicone/vinyl dimethicone crosspolymer, biosaccharide gum-1, oxothiazolidinecarboxylic acid, ascorbic acid, sodium styrene/maleic anhydride copolymer, salicylic acid, cyclohexasiloxane, hydrogenated polyisobutene, biosaccharide gum-1 and sodium levulinate and glyceryl caprylate and sodium anisate, lemon extract, alcohol and Gentiana lutea root extract, and dimethicone and polyethylene glycol/polypropylene glycol-18/18 dimethicone.

In some embodiments, the iontophoresis composition includes one or more emollients selected from C12-15 alkyl benzoate, caprylyl glycol, glyceryl stearate and polyethylene glycol 100 Stearate, ethylhexyl palmitate, dimethicone and dimethiconol, dimethicone, dimethicone and dimethicone/vinyl dimethicone crosspolymer, cyclohexasiloxane, hydrogenated polyisobutene, biosaccharide gum-1 and sodium levulinate and glyceryl caprylate and sodium anisate, and dimethicone and polyethylene glycol/polypropylene glycol-18/18 dimethicone.

In some embodiments, the iontophoresis composition includes one or more emulsifiers selected from glyceryl stearate and polyethylene glycol 100 Stearate, cetyl alcohol, xanthan gum, triethanolamine, biosaccharide gum-1 and sodium levulinate and glyceryl caprylate and sodium anisate, and dimethicone and polyethylene glycol/polypropylene glycol-18/18 dimethicone.

In some embodiments, the iontophoresis composition includes one or more cleansing agents selected from glyceryl stearate and polyethylene glycol 100 Stearate.

In some embodiments, the iontophoresis composition includes one or more stabilizers selected from cetyl alcohol, xanthan gum, ammonium polyacryldimethyltauramide, sodium styrene/maleic anhydride copolymer, carbomer, and acrylates/C10-30 alkylacrylate crosspolymer.

In some embodiments, the iontophoresis composition includes one or more viscosity increasers selected from cetyl alcohol, xanthan gum, dimethicone and dimethicone/vinyl dimethicone crosspolymer, carbomer, and acrylates/C10-30 alkylacrylate crosspolymer.

In some embodiments, the iontophoresis composition includes one or more antioxidants selected from tocopheryl acetate, and ascorbic acid.

In some embodiments, the iontophoresis composition includes one or more binders selected from xanthan gum.

In some embodiments, the iontophoresis composition includes one or more skin bleaching agents selected from oxothiazolidinecarboxylic acid.

In some embodiments, the iontophoresis composition includes one or more pH adjusters selected from triethanolamine, potassium hydroxide, and sodium hydroxide.

In some embodiments, the iontophoresis composition includes one or more buffering agents selected from potassium hydroxide and hydroxyethylpiperazine ethane sulfonic acid, and sodium hydroxide.

In some embodiments, the iontophoresis composition includes one or more denaturants selected from sodium hydroxide.

In some embodiments, the iontophoresis composition includes one or more bulking agents selected from nylon-12.

In some embodiments, the iontophoresis composition includes one or more opacifying agents selected from nylon-12.

### EXAMPLES

Iontophoretic transport experiments were conducted to quantify skin deposition of ascorbic acid (vitamin C) with different formulations.

Kinetics of ascorbic acid prepared in different formulations containing 5% of ascorbic acid and anionic and non-ionic polymers at specific ratios in a mixture of polar solvents, and their active's release in vitro subjected to 0.2 mA/cm2 cathodal (-) iontophoresis during different timing points were measured.

The skin delivery of acid ascorbic from different cosmetic gel formulations developed specially for iontophoresis (without the presence of ionized preservatives and chelating agents) containing anionic and neutral polymers was investigated, in order to assess their efficacy on active agent's release.

In the following, the active agent is Vitamin C (acid ascorbic).

As a second step, the formulations including different polar solvents (glycols and ethanol) were evaluated in order to optimized delivery of the active agent.

Two control composition (outside the invention) have been used as controls: composition 1 (o/w) 5% ascorbic acid (positive control, sensitive to iontophoresis), and composition 2 (w/o) inverted siliconized 7% ascorbic acid (negative control, inadequate for iontophoresis).

### 1 - Materials & Methods

The formulations were tested in vitro using Franz diffusion cells at 0.2 mA/cm2 using Ag/AgCl electrodes and DC current for 10 and 20 min. Such formulations were tested in different vitro studies.

The purpose of the first study was to evaluate the effect of iontophoresis on the skin deposition of active agent from applying the current (0.2 mA/cm²) for 5 minutes and afterwards, 60 min of passive diffusion (w/o iontophoresis). The formulations tested were: 5% active agent (compositions 3, 4, 5 and 6) vs. composition 1 with 5% and composition 2 containing 7% active agent.

The second study was performed applying the current (0.2 mA/cm²) for 10 or 20 minutes with no subsequent passive diffusion period. The compositions evaluated were: 5% active agent (compositions 3, 6 and 7) and the control composition 1.

The third study conducted applying the current (0.2 mA/cm²) for 10 or 20 minutes with "no subsequent passive diffusion" period. The compositions evaluated contained different polar solvents as enhancers: 5% active agent (compositions 8, 9, 10 and 11) and the the control composition 1.

All studies included control(s), which was performed using the same set-up as for iontophoresis but without current application.

The specific rate of each ingredient of the formulations developed is described in the table below.

| **Ingredients/Composition** | **Reference/Company** | **1*** | **6** | **3** | **7** | **4** | **5** | **8** | **10** | **11** |
|---|---|---|---|---|---|---|---|---|---|---|
| POTASSIUM HYDROXIDE | | 2,86 | | | | | | 2,7 | 2,7 | 2,7 |
| SODIUM HYDROXIDE | | 0,6 | 3,0** | 3,0** | 1,0 | 3,0* | 3,0** | | | |
| PHENOXYETHANOL | | 0,7 | 0,7 | 0,7 | 0,7 | 0,7 | 0,7 | 0,7 | 0,7 | 0,7 |
| HYDROXYPROPYL METHYLCELLULOSE **(HPMC)** | METHOCEL F 4 M PERSONAL CARE GRADE of company DOW CHEMICAL | | 1,0 | | | | | 1,0 | | |
| AMMONIUM POLYACRYLOYLDIME THYLTAURATE **(AMPS-amonium)** | HOSTACERIN AMPS of company CLARIANT | | | 1,0 | | | | | 1,0 | |
| SODIUM ACRYLOYLDIMETHYLT AURATE/VP CROSSPOLYMER **(Aristoflex AVS)** | ARISTOFLEX AVS of company CLARIANT | | | | 1,0 | | | | | 1,0 |
| sodium carboxymethyl cellulose purified (CELLULOSE GUM) | AQUASORBA A 500 of company ASHLAND | | | | | 1,0 | 0,5 | | | |
| **BUTYLENE GLYCOL** | | | 20,0 | 20,0 | 20,0 | 20,0 | 20,0 | | | |
| **ALCOHOL DENAT.** | | | | | | | | 10,0 | 10,0 | 10,0 |
| **PROPYLENE GLYCOL** | | | | | | | | 20,0 | 20,0 | 20,0 |
| **ASCORBIC ACID** | | 5,0 | 5,0 | 5,0 | 5,0 | 5,0 | 5,0 | 5,0 | 5,0 | 5,0 |
| WATER | | 46 | 70,3 | 70,3 | 72,3 | 70,3 | 70,8 | 60,6 | 60,6 | 60,6 |
| **GLYCERIN** | | 7 | | | | | | | | |
| CYCLOHEXASILOXANE **(SILICON BASED POLYMER)** | | 3 | | | | | | | | |
| **ACRYLATES/C10-30** ALKYL ACRILATE CROSSPOLYMER | | 0,4 | | | | | | | | |
| BIOSACCHARIDE **GUM** | | 3 | | | | | | | | |
| SODIUM STYRENE/MA COPOLYMER **(SMA)** | | 2,5 | | | | | | | | |

| | | | | | | | | | | |
|---|---|---|---|---|---|---|---|---|---|---|
| * Skin renew formulation contain further ingredients, the mentioned in the table are the selected ones that have more relevancy for the study ** Concentration of NaOH at 30% | | | | | | | | | | |

### 2 - Results

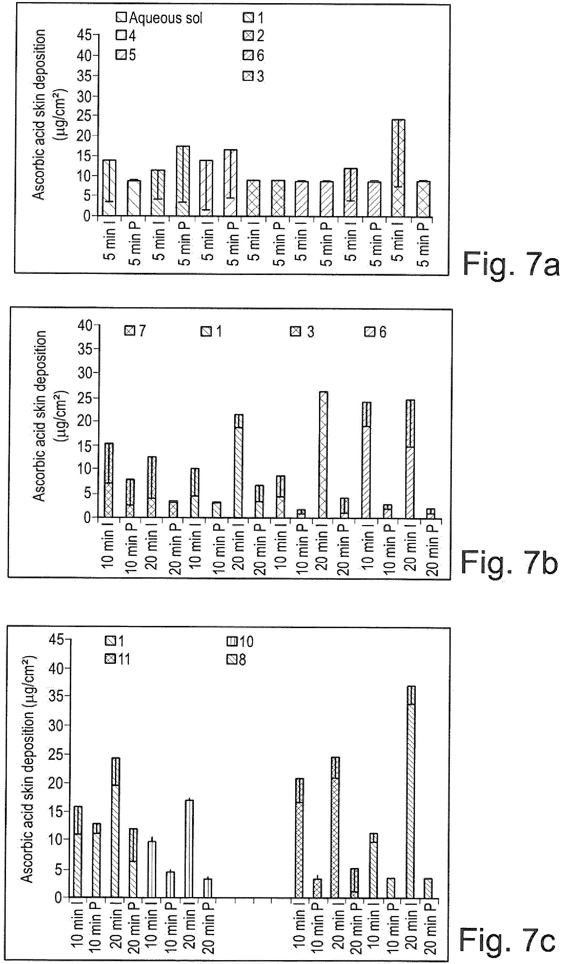

The results are shown on figure 7a to 7c. I means that iontophoresis treatment was applied and P that treatment was passive.

The results of the first preliminary experiment are shown on figure 7a, and those revealed few statistically significant differences between the formulations. The absence of significant increases when using iontophoresis was due to its short duration (5 min) with respect to the passive delivery phase (60 min), the presence of competing ions and the complex nature of the formulations. Statistical analysis showed that with respect to the iontophoresis results, delivery of active agent from (i) composition 5 (1% AMPS-amonium) was significantly superior to that from the control compositions 1 and 2 (p<0.05; ANOVA, Student Newman Keuls test).

In the second experiment, shown on figure 7b, two scales on ascorbic acid diffusion into the skin with iontophoresis can be defined at 10 and 20 min:
At 10 minutes, composition 6 (containing HPMC) is much better than composition 3 (AMPS-amonium), which is itself equivalent to composition 1 (SMA) and composition 7 (Aristoflex AVS).

At 20 minutes, composition 6 (containing HPMC) is equivalent to composition 3 (AMPS-amonium), which is greater than composition 1 (SMA), which is itself greater than composition 7 (Aristoflex AVS).

Composition 6 containing HPMC (neutral polymer) showed the greatest deposition after iontophoresis for 20 min compared to the composition 3 containing the anionic polymers (AMPS-amonium) and the composition 7 (Aristoflex AVS).

From the third experiment, whose results are shown on figure 7c, propylene glycol and ethanol are added as polar solvents. According to the iontophoretic duration, two different scales on ascorbic acid deposition into the skin with iontophoresis can be established:
At 10 minutes, Composition 11 has better results than Composition 8, which itself is similar to Composition 10.

At 20 minutes, Composition 8 has much better results than Composition 11.

From the results, Composition 8 containing HPMC (neutral polymer), 10% alcohol and 20% propylene glycol showed the greatest deposition after iontophoresis for 20 min.

Enhancement ratio (Diffusion by iontophoresis / passive diffusion) can be calculated for this last experiment due to the relative low variability. The results are shown in the table below.

| **Ascorbic acid skin deposition (µg/cm²)** | | | | |
|---|---|---|---|---|
| **Composition** | **Condition** | **Mean** | **SD** | **Enhancement ratio (Ionto/Passive)** |
| **1** | 10 min I | 15.9 | 4.9 | 1.2 |
| | 10 min P | 12.8 | 1.4 | |
| | 20 min I | 24.2 | 4.7 | 2.0 |
| | 20 min P | 11.9 | 5.7 | |
| **10** | 10 min I | 9.8 | 5.2 | 2.1 |
| | 10 min P | 4.7 | 3.1 | |
| | 20 min I | 17.3 | 4.7 | 5.1 |
| | 20 min P | 3.4 | 0.0 | |
| **11** | 10 min I | 20.8 | 4.4 | 6.1 |
| | 10 min P | 3.4 | 0.0 | |
| | 20 min I | 24.6 | 3.5 | 4.9 |
| | 20 min P | 5.0 | 3.9 | |
| **8** | 10 min I | 11.5 | 1.9 | 3.4 |
| | 10 min P | 3.4 | 0.0 | |
| | 20 min I | 37.1 | 3.3 | 10.9 |
| | 20 min P | 3.4 | 0.0 | |

The compositions 8, 10 and 11 have much better results than the control composition 1.

From the formulations studied, composition 8 showed the greatest improvement as evidenced by the highest enhancement ratio. ER =10.9 seen after iontophoresis for 20 min leading to greatest ascorbic acid diffusion into the skin.

The use of anionic polymer AMPs formulated in simple gels show an improvement of active agent iontophoretic delivery compare to the passive diffusion of the active agent. However, their effect is not superior to that found with the neutral polymer HPMC of composition 8.

### 3 - Conclusion

The results show the optimization of 5% Vitamin C formulation dedicated to iontophoresis to limit the risks of exposure to other cosmetic ingredients usually contained in topical formulations.

While illustrative embodiments have been illustrated and described, it will be appreciated that various changes can be made therein without departing from the spirit and scope of the claimed subject matter.

## Claims

1. A iontophoresis method of delivering Vitamin C through the skin, the iontophoresis method comprising:
applying to the skin of a biological subject a composition comprising one or more of Vitamin C, Vitamin C derivatives, ions of Vitamin C, and ions of Vitamin C derivatives, and at least an anionic or non-ionic polymer,
applying simultaneously, successively or sequentially over time a selected current profile, either continuous direct current, pulsed current or a combination of both, from any device and/or support comprising at least one electrode to the skin, the continuous direct current, the pulsed current or the combination of both of a character and for a duration sufficient to transdermally deliver Vitamin C to the biological subject, and transporting different rates of Vitamin C across the skin in accordance to the selected current mode.

2. The iontophoresis method of the preceding claim, wherein applying a selected current profile to a biological subject includes generating a continuous direct current stimulus having an average current density ranging from 0.001 mA/cm² to 0.5 mA/cm², preferably from 0.01 mA/cm² to 0.4 mA/cm², and more preferably from 0.05 mA/cm² to 0.3 mA/cm², and in particular of 0.2 mA/cm².

3. The iontophoresis method of the preceding claim, wherein the continuous direct current stimulus is applied for a duration ranging from 30 seconds to 120 minutes, preferably from 2 minutes to 50 minutes and more preferably from 3 minute to 40 minutes.

4. The iontophoresis method of any preceding claim, wherein the composition comprises one or more of Vitamin C, Vitamin C derivatives, ions of Vitamin C, and ions of Vitamin C derivatives present in amounts ranging from 0.01 % to 100 % by weight, preferably from 0.5 % to 60 % by weight.

5. The iontophoresis method of any preceding claim, wherein the composition comprises one or more of Vitamin C, Vitamin C derivatives, ions of Vitamin C, and ions of Vitamin C derivatives present in an amount of 5 % by weight.

6. The iontophoresis method according to any preceding claims, comprising the step of measuring at least one of the temperature of the skin, the impedance of the skin, and a pH of the composition, and wherein the application of current profile may be reduced to a safety level when a measured value measured by one of the sensors exceeds a safety range or a safety value.

7. The iontophoresis method of any preceding claim, the composition further comprising one or more anionic or non-ionic polymers present in amounts ranging from 0.01 % to 10 % by weight.

8. The iontophoresis method of any preceding claim, the one or more anionic or non-ionic polymers having a molecular weight ranging from 100 to 5,000,000 Daltons, preferably from 500 to 3,000,000 Daltons.

9. The iontophoresis method of any preceding claim, wherein the composition comprises hydroxypropyl methyl cellulose (HPMC) and/or ammonium polyacryloyldimethyl taurate and/or sodium acryloyldimethyltaurate/VP crosspolymer.

10. A cosmetic composition, comprising one or more of Vitamin C, Vitamin C derivatives, ions of Vitamin C, and ions of Vitamin C derivatives, and at least an anionic or non-ionic polymer.

11. The cosmetic composition of the preceding claim, comprising the anionic or non-ionic polymer in an amount ranging from 0.01% to 10 % by weight, preferably of about 1.0 % by weight, relative to the total weight of the composition.

12. The cosmetic composition of the two preceding claim, comprising at least one of hydroxypropyl methyl cellulose (HPMC) and/or ammonium polyacryloyldimethyl taurate and/or sodium acryloyldimethyltaurate/VP crosspolymer.

13. A iontophoresis kit comprising:
- An iontophoresis composition including one or more of Vitamin C, Vitamin C derivatives, ions of Vitamin C, and ions of Vitamin C derivatives, and at least an anionic or non-ionic polymer, and
- An iontophoresis device for carrying the iontophoresis method of anyone of claims 1 to 10.

14. A iontophoresis kit comprising:
- An iontophoresis composition including one or more of Vitamin C, Vitamin C derivatives, ions of Vitamin C, and ions of Vitamin C derivatives, and at least an anionic or non-ionic polymer, and
- An iontophoresis device for delivering the iontophoresis composition through the skin, configured for applying a selected current profile, either continuous direct current, pulsed current or a combination of both.

15. The iontophoresis kit of the two preceding claim, wherein the composition is an aqueous composition.

16. The iontophoresis kit of any one of the three preceding claims, the kit being configured such that Vitamin C and water are already mixed in the composition when the composition is applied to the skin.

17. The iontophoresis kit of any one of claims 12 to 16, the device comprising at least one of a temperature sensor, an impedance sensor, and a pH sensor, and wherein the device may be configured such that the application of current profile is reduced to a safety level when a measured value measured by one of the sensors exceeds a safety range or a safety value.
